(19) Europäisches Patentamt
European Patent Office
Office européen des brevets

(11) EP 1 935 399 A2

(12) DEMANDE DE BREVET EUROPEEN

(43) Date de publication:
**25.06.2008 Bulletin 2008/26**

(21) Numéro de dépôt: **07123413.2**

(22) Date de dépôt: **17.12.2007**

(51) Int Cl.:
***A61K 8/73*** *(2006.01)* ***A61K 8/81*** *(2006.01)*
***A61K 8/891*** *(2006.01)* ***A61K 8/895*** *(2006.01)*
***A61Q 19/00*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **20.12.2006 FR 0655682**

(71) Demandeur: **L'ORÉAL**
**75008 Paris (FR)**

(72) Inventeurs:
- **L'Alloret, Florence**
**75014, PARIS (FR)**
- **Millecamps, Danielle**
**91270, VIGNEUX SUR SEINE (FR)**

(74) Mandataire: **Le Coupanec, Pascale A.M.P.**
**Nony & Associés,**
**3 rue de Penthièvre**
**75008 Paris (FR)**

# (54) Produit cosmétique comprenant des composés siliconés et un polymère amphiphile

(57) La présente invention concerne un kit cosmétique utile pour le soin et/ou le maquillage de matière(s) kératinique(s), notamment de la peau, comprenant au moins deux compositions différentes et conditionnées séparément avec l'une au moins des compositions étant de type émulsion et comprenant au moins un polymère amphiphile, le kit comprenant un ou plusieurs composés X, un ou plusieurs composés Y, et au moins un catalyseur, avec au moins un des composés X et Y étant siliconé et lesdits composés X et Y étant susceptibles de réagir ensembles par une réaction d'hydrosylilation en présence d'un catalyseur, lorsqu'ils sont mis en contact les uns avec les autres en présence d'un catalyseur, et dans lequel les composés X, Y et le catalyseur ne sont pas présents simultanément dans la même composition

EP 1 935 399 A2

## Description

La présente invention a pour objet principal un kit cosmétique, notamment de maquillage et/ou de soin des matières kératiniques comprenant une première composition contenant au moins un composé X et une seconde composition contenant au moins un composé Y, les composés X et Y étant aptes à réagir ensemble le cas échéant en présence d'un catalyseur ou d'un peroxyde, et l'un au moins des composés étant siliconé avec au moins l'une des compositions contenant en outre un polymère amphiphile.

Le kit selon l'invention peut être un produit de maquillage et/ou de soin des matières kératiniques dont les fibres kératiniques, en particulier de la peau, des lèvres, des cils, des sourcils ou des ongles.

Dans le domaine des maquillages et/ou des soins cosmétiques un grand nombre de compositions se présente sous la forme d'émulsions. Ces émulsions contiennent généralement un ou plusieurs émulsionnant(s) choisi(s) parmi les émulsionnants amphotères, anioniques, cationiques ou non ioniques, utilisés seuls ou en mélange, et éventuellement un co-émulsionnant.

Les émulsionnants sont choisis de manière appropriée suivant l'émulsion à obtenir (E/H ou H/E).

Parmi les émulsionnants susceptibles d'être mis en oeuvre, on peut plus particulièrement citer les polymères amphiphiles qui sont notamment appréciés dans le domaine de la cosmétique au regard des performances qui leur sont associées.

Ainsi, les émulsions stabilisées par des polymères amphiphiles sont particulièrement stables et conduisent à des propriétés de texture novatrices par rapport aux émulsions stabilisées par des tensioactifs de faible masse molaire. Ces émulsions peuvent comprendre par ailleurs des natures de phases grasses très variées : triglycérides, alcanes, esters, silicones, filtres solaires, perfluorés, soit seules soit en mélange. Enfin, le rapport entre la concentration en polymères amphiphiles et le taux de phase dispersée est faible (environ 0,1) par rapport au cas des émulsions stabilisées par des tensioactifs (environ 0,4).

Toutefois, après application sur la peau, la phase grasse de ces émulsions peut conduire à une peau relativement brillante, cet effet est amplifié avec le taux d'huile et avec la présence d'huile non volatile.

Or, si ces propriétés de brillance sont recherchées pour certains produits cosmétiques à l'image des rouges à lèvres et des vernis à ongles, elles peuvent en revanche s'avérer indésirables pour d'autres produits cosmétiques à l'image des fonds de teint et des produits de soin.

En conséquence, la présente invention vise précisément à proposer des produits cosmétiques de maquillage et/ou de soin incorporant des polymères amphiphiles mais en revanche dotés d'effet brillant amoindri voire dénués d'effet brillant.

De manière inattendue, les inventeurs ont découvert qu'il était possible de donner satisfaction en ces termes sous réserve de formuler les émulsions cosmétiques considérées avec un système de polymères spécifiques.

Ce système met en présence deux composés notamment siliconé, qui lorsqu'ils sont en contact, le cas échéant en présence d'un catalyseur ou d'un peroxyde, polymérisent *in situ* à pression atmosphérique et température ambiante, pour former des films avantageusement biocompatibles et non collants. De tels systèmes sont notamment en partie décrits dans les documents WO 01/96450 et GB 2 407 496.

Ces films polymériques, susceptibles d'être formés *in situ* sur un support, notamment de type matière kératinique, s'avèrent dotés de propriétés avantageuses en terme de cosmétique, à savoir bonne adhésion, bonne tenue et confort.

Ainsi, selon un premier aspect, la présente invention a pour objet un kit cosmétique utile pour le soin et/ou le maquillage de matière(s) kératinique(s), notamment de la peau, comprenant au moins deux compositions différentes et conditionnées séparément avec l'une au moins des compositions étant de type émulsion et comprenant au moins un polymère amphiphile, le kit comprenant un ou plusieurs composés X, un ou plusieurs composés Y, et éventuellement au moins un catalyseur ou un peroxyde, avec au moins un des composés X et Y étant siliconé et lesdits composés X et Y étant susceptibles de réagir ensembles par une réaction d'hydrosylilation en présence d'un catalyseur, ou par une réaction de condensation, ou par une réaction de réticulation en présence d'un peroxyde, lorsqu'ils sont mis en contact les uns avec les autres, le cas échéant en présence d'un catalyseur ou d'un peroxyde, et dans lequel les composés X, Y et le catalyseur ou le peroxyde lorsqu'il est présent, ne sont pas présents simultanément dans la même composition.

Selon un mode de réalisation particulier, le kit comprend au moins :

i. une première composition contenant, dans un milieu physiologiquement acceptable, au moins un composé X et
ii. une seconde composition contenant, dans un milieu physiologiquement acceptable, au moins un composé Y,
avec l'une au moins desdites première et seconde compositions étant sous la forme d'une émulsion et contenant au moins un polymère amphiphile, et l'une au moins desdites première et seconde compositions contenant en outre le cas échéant au moins un catalyseur ou un peroxyde.

Selon une variante de réalisation, les première et seconde compositions sont toutes deux des émulsions

stabilisées respectivement par au moins un polymère amphiphile.

Lors du mélange des compositions cosmétiques les contenant, les composés X et Y polymérisent *in situ,* adhérent aux matières kératiniques et permettent d'obtenir des dépôts moins brillants sur la peau.

De préférence, la première composition comprenant le composé X et la seconde composition comprenant le composé Y sont conditionnées dans des conditionnements séparés.

Par exemple, chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon. Chaque composition peut aussi peut être conditionnée dans un compartiment au sein d'un même article conditionnement, le mélange des deux compositions s'effectuant à la ou les extrémités de l'article de conditionnement lors de la délivrance de chaque composition.

Alternativement, chacune des première et seconde compositions peut être conditionnée dans un article de conditionnement différent.

L'invention concerne également un procédé de soin cosmétique et/ou de maquillage de matière(s) kératinique (s) comprenant au moins l'application sur les matières kératiniques (a) d'au moins un polymère amphiphile, (b) d'un ou plusieurs composés X, (c) d'un ou plusieurs composés Y, avec au moins un des composés X et Y étant siliconé et lesdits composés X et Y étant susceptibles de réagir ensemble par une réaction d'hydrosylilation en présence d'un catalyseur, ou par une réaction de condensation, ou par une réaction de réticulation en présence d'un peroxyde, lorsqu'ils sont mis en contact l'un avec l'autre, et (d) le cas échéant d'au moins un catalyseur ou un peroxyde, les applications (a), (b), (c) et (d) pouvant être simultanée ou séquencée selon un ordre indifférent sous réserve qu'il soit propice à l'interaction desdits composés X et Y.

Ainsi, le ou les composés X, le ou les composés Y, peuvent être appliqués sur les matières kératiniques à partir de plusieurs compositions dont au moins une sous forme d'émulsion, les compositions contenant respectivement le ou les composés X, le ou les composés Y, le ou les polymères amphiphiles, seuls ou en mélange, ou à partir d'une seule composition contenant le ou les composés X, le ou les composés Y, le ou les polymères amphiphiles.

Selon un mode de réalisation particulier de l'invention, on applique sur les fibres kératiniques une première composition comprenant au moins le ou les composés X, et une seconde composition contenant au moins le ou les composés Y, avec l'une au moins desdites première et seconde compositions étant sous forme d'une émulsion et contenant en outre au moins un polymère amphiphile, l'une au moins des première et seconde compositions contenant en outre le cas échéant au moins un catalyseur ou un peroxyde.

Plus particulièrement, ledit procédé peut consister à appliquer sur lesdites matières kératiniques au moins une composition de type émulsion comprenant, dans un milieu physiologiquement acceptable, au moins un composé X, au moins un composé Y, le cas échéant, au moins un catalyseur ou un péroxyde et au moins un polymère amphiphile.

L'invention concerne également un procédé cosmétique de maquillage et/ou de soin des matières kératiniques notamment de la peau humaine, comprenant l'application sur lesdites matières kératiniques :

- d'au moins une couche d'une première composition contenant, dans un milieu physiologiquement acceptable, au moins un composé X ;
- d'au moins une couche d'une seconde composition contenant, dans un milieu physiologiquement acceptable, au moins un composé Y,
  l'un au moins des composés X et Y étant un composé siliconé, lesdits composés X et Y étant susceptibles de réagir ensemble par une réaction d'hydrosylilation en présence d'un catalyseur, ou par une réaction de condensation, ou par une réaction de réticulation en présence d'un péroxyde, lorsqu'ils sont mis en contact l'un avec l'autre, avec l'une au moins desdites première et seconde compositions étant une émulsion et comprenant au moins un polymère amphiphile, et
  l'une au moins des première et seconde compositions contenant en outre le cas échéant au moins un catalyseur ou un peroxyde.

Selon une variante, le procédé consiste à appliquer sur les matières kératiniques au moins une couche de la seconde composition comprenant le composé Y et le cas échéant un polymère amphiphile, puis à déposer sur la ou les couches de ladite seconde composition au moins une couche de la première composition comprenant le composé X et le cas échéant un polymère amphiphile, l'une au moins des première et seconde compositions contenant en outre le cas échéant au moins un catalyseur ou un péroxyde.

Selon une autre variante de ce procédé, il est possible d'appliquer sur les matières kératiniques au moins une couche de la première composition comprenant le composé X et le cas échéant au moins un polymère amphiphile, puis de déposer sur la ou les couches de ladite première composition au moins une couche de la seconde composition comprenant le composé Y et le cas échéant au moins un polymère amphiphile, l'une au moins des première et seconde compositions contenant en outre le cas échéant au moins un catalyseur ou un péroxyde.

On peut également appliquer en alternance sur les matières kératiniques plusieurs couches de chacune des première et seconde compositions.

La composition appliquée peut être également obtenue en mélangeant de façon extemporanée une première composition contenant au moins le composé X et une seconde composition contenant au moins le composé Y, l'une au moins des première et seconde compositions étant une émulsion et comprenant au moins un polymère amphiphile, et l'une au moins des première et seconde compositions contenant en outre le cas échéant au moins un catalyseur ou un péroxyde.

Au sens de l'invention, notamment dans le mode de réalisation où la composition est obtenue comme décrit ci-dessus, à savoir en mélangeant de façon extemporanée une première composition contenant au moins le composé X et une seconde composition contenant au moins le composé Y, il est entendu que le mélange ainsi formé comprend des composés X et/ou Y sous une forme n'ayant pas encore réagi et non pas exclusivement sous la forme de leur produit de réaction par hydrosilylation, par polycondensation et/ou par réticulation en présence d'un peroxyde.

Ainsi, la formation du produit de réaction selon l'invention, peut être soit réalisée directement sur la surface de la matière kératinique devant être traitée, soit initiée juste avant application par mélange extemporané des composés X et Y dans des conditions propices à leur interaction, la formation du produit de réaction étant dans ce dernier cas finalisée en surface de la matière kératinique.

Pour des raisons évidentes, et au regard de la grande réactivité des composés X et/ou Y, il est en effet nécessaire que leur mise en oeuvre soit réalisée dans des conditions propices à la maniabilité de la composition le (ou les) contenant notamment en vue de son étalement par exemple. Le procédé conforme à l'invention met donc en oeuvre une composition contenant des composés X et Y, et donc non figée sous la forme du film final attendu et résultant de la réaction de la totalité de X et/ou de la totalité de Y.

Selon un mode de réalisation, le procédé comprend une étape supplémentaire consistant à déposer sur la ou les couches de compositions comprenant X et Y, au moins une couche d'une troisième composition comprenant un polymère filmogène et un milieu solvant organique ou aqueux.

Selon une autre variante, la composition appliquée contient au moins un des composés X et Y sous une forme encapsulée.

Ainsi, la présente invention vise également une composition cosmétique, notamment de soin et/ou de maquillage de matières kératiniques, de type émulsion, contenant, dans un milieu physiologiquement acceptable, au moins un composé X, un composé Y et le cas échéant au moins le catalyseur et le peroxyde si nécessaire à l'interaction desdits composés X et Y et au moins un polymère amphiphile tels que définis ci-dessous avec au moins un des composés X et Y étant sous une forme encapsulée.Selon une variante de réalisation préférée de ce mode, les deux composés X et Y sont présents sous des formes encapsulées séparées.

Selon ce mode de réalisation, les deux composés X et Y peuvent être conditionnés dans une même composition tout en s'affranchissant du risque de réaction prématurée entre eux. Cette réaction n'intervient qu'au moment où la composition est manipulée préalablement ou au moment de son application sur la matière kératinique. La
ou les forme(s) encapsulée(s) du composé X et/ou Y se brisent au séchage et les composés X et Y mis alors en contact, réagissent pour former le film attendu.

**COMPOSES X ET Y**

Par composé siliconé, on entend un composé polyorganosiloxane, c'est-à-dire comprenant au moins deux unités organosiloxanes, par exemple au moins 5 unités organosiloxane, notamment au moins 10 unités organosiloxane. Selon un mode de réalisation particulier, l'un au moins des composés X et Y, ou les composés X et les composés Y sont siliconés. Les composés X et Y peuvent être aminés ou non aminés.

Selon un autre mode de réalisation, au moins un des composés X et Y est un polymère dont la chaîne principale est formée majoritairement d'unités organosiloxanes. Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites "room temperature vulcanization".

Les composés X et Y peuvent réagir ensemble à une température variant entre la température ambiante et 180°C. Avantageusement les composés X et Y sont susceptibles de réagir ensemble à température ambiante (20 ± 5°C) et pression atmosphérique, ou avantageusement en présence d'un catalyseur, par une réaction d'hydrosilylation ou une réaction de condensation, ou une réaction de réticulation en présence d'un peroxyde.

Groupes polaires

Selon un mode de réalisation particulier, l'un au moins des composé X et Y, par exemple le composé X, est

porteur d'au moins un groupe polaire susceptible de former au moins une liaison hydrogène avec les matières kératiniques.

Par groupe polaire, on entend un groupe comportant des atomes de carbone et d'hydrogène dans sa structure chimique et au moins un hétéroatome (tel que O, N, S et P), tel que ledit groupe est apte à établir au moins une liaison hydrogène avec les matières kératiniques.

Des composés porteurs d'au moins un groupement apte à établir une liaison hydrogène sont particulièrement avantageux, car ils apportent aux compositions les contenant une meilleure adhérence sur les matières kératiniques.

Le ou les groupe(s) polaire(s) porté(s) par au moins l'un des composés X et Y est/sont apte(s) à établir une liaison hydrogène, et comporte(nt) soit un atome d'hydrogène lié à un atome électronégatif, soit un atome électronégatif comme par exemple l'atome d'oxygène, d'azote ou de soufre. Lorsque le groupe comporte un atome d'hydrogène lié à un atome électronégatif, l'atome d'hydrogène peut interagir avec un autre atome électronégatif porté, par exemple par une autre molécule, telle que la kératine, pour former une liaison hydrogène. Lorsque le groupement comporte un atome électronégatif, l'atome électronégatif peut interagir avec un atome d'hydrogène lié à un atome électronégatif porté, par exemple par une autre molécule, telle que la kératine, pour former une liaison hydrogène.

Avantageusement, ces groupes polaires peuvent être choisis parmi les groupes suivants :

- acides carboxyliques -COOH,
- alcools, tels que : $-CH_2OH$ ou -CH(R)OH, R étant un radial alkyle comprenant de 1 à 6 atomes de carbone,
- amino de formule $-NR_1R_2$, dans laquelle les $R_1$ et $R_2$ identiques ou différents représentent un radial alkyle comprenant de 1 à 6 atomes de carbone ou l'un des $R_1$ ou $R_2$ désigne un atome d'hydrogène, et l'autre des $R_1$ et $R_2$ représente un radical alkyle comprenant de 1 à 6 atomes de carbone,
- pyridino,
- amido de formule -NH-COR' ou -CO-NH-R' dans laquelle R' représente un atome d'hydrogène ou un radial alkyle comprenant de 1 à 6 atomes de carbone,
- pyrrolidino choisi de préférence parmi les groupes de formule :

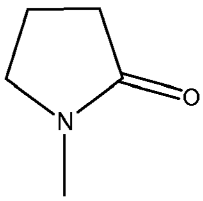 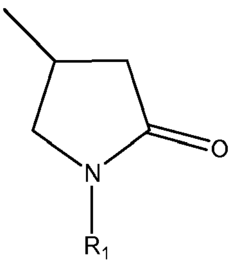 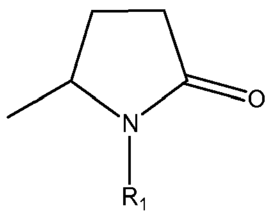 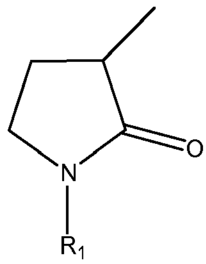

$R_1$ étant un radial alkyle comprenant de 1 à 6 atomes de carbone,
- carbamoyle de formule -O-CO-NH-R' ou -NH-CO-OR', R' étant tel que défini ci-dessus,
- thiocarbamoyle tel que -O-CS-NH- R' ou -NH-CS-OR', R' étant tel que défini ci-dessus,
- uréyle tel que $-NR'-CO-N(R')_2$, les groupes R' identiques ou différents étant tels que définis ci-dessus,
- sulfonamido tel que $-NR'-S(=O)_2-R'$, R' répondant à la définition ci-dessus.

De préférence, ces groupes polaires sont présents à une teneur inférieure ou égale à 10 % en poids par rapport au poids de chaque composé X ou Y, de préférence inférieure ou égale à 5 % en poids, par exemple en une teneur allant de 1 à 3 % en poids.

Le ou les groupes polaires peu(ven)t être situé(s) dans la chaîne principale du composé X et/ou Y ou peuvent être pendants à la chaîne principale ou situés aux extrémités de la chaîne principale du composé X et/ou Y.

## 1- Composés X et Y susceptibles de réagir par hydrosilylation

Selon un mode de réalisation, l'invention concerne un kit cosmétique utile pour le soin et/ou le maquillage de matière(s) kératinique(s), notamment de la peau, comprenant au moins deux compositions différentes et conditionnées séparément avec l'une au moins des compositions étant de type émulsion et comprenant au moins un polymère amphiphile, le kit comprenant un ou plusieurs composés X, un ou plusieurs composés Y, et au moins un catalyseur, avec au moins un des composés X et Y étant siliconé et lesdits composés X et Y étant susceptibles de réagir ensemble par une réaction d'hydrosilylation lorsqu'ils sont mis en contact les uns avec les autres en présence d'un catalyseur, et dans lequel les composés X, Y et le catalyseur ne sont pas présents simultanément dans la même composition.

Selon ce mode de réalisation, les composés X et Y sont susceptibles de réagir par hydrosilylation en présence d'un catalyseur, cette réaction pouvant être de manière simplifiée schématisée comme suit :

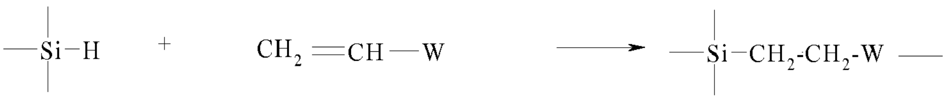

avec W représentant une chaîne carbonée et/ou siliconée contenant un ou plusieurs groupements aliphatiques insaturés.
Dans ce cas, le composé X peut être choisi parmi les composés siliconés comprenant au moins deux groupements aliphatiques insaturés. A titre d'exemple, le composé X peut être un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.
Selon un mode de réalisation, le composé X et/ou le composé Y est porteur d'au moins un groupe polaire, tel que décrit ci-dessus, susceptible de former au moins une liaison hydrogène avec les matières kératiniques. Ce groupe polaire est avantageusement porté par le composé X qui comprend au moins deux groupements aliphatiques insaturés.
Selon un mode de réalisation, le composé X est choisi parmi les polyorganosiloxanes comprenant au moins deux groupements aliphatique insaturés, par exemple deux ou trois groupements vinyliques ou allyliques, liés chacun à un atome de silicium.
Selon un mode de réalisation avantageux, le composé X est choisi parmi les polyorganosiloxanes comprenant des unités siloxanes de formule :

$$R_mR'SiO_{\frac{(3-m)}{2}} \qquad (I)$$

dans laquelle :

- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :

  o un groupement hydrocarboné aliphatique insaturé comprenant de 2 à 10, de préférence de 3 à 5 atomes de carbone comme par exemple un groupe vinyle ou un groupe -R''-CH=CHR''' dans lequel R'' est une chaîne hydrocarbonée aliphatique divalente, comprenant de 1 à 8 atomes de carbone, liée à l'atome de silicium et R''' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
  o un groupement hydrocarboné cyclique insaturé comprenant de 5 à 8 atomes de carbone comme par exemple un groupe cyclohexènyle.

De préférence R' est un groupement hydrocarboné aliphatique insaturé, de préférence un groupe vinyle.
Selon un mode de réalisation, R représente un radical alkyle comprenant de 1 à 10 atomes de carbone ou encore un groupement phényle, et de préférence un radical méthyle, et R' est un groupe vinyle.
Selon un mode de réalisation particulier, le polyorganosiloxane comprend également des unités de formule :

$$R_nSiO_{\frac{(4-n)}{2}} \qquad (II)$$

dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé Y par hydrosilylation en présence d'un catalyseur. On peut citer par exemple les résines de type MQ ou MT portant elle-même des extrémités réactives insaturées $-CH=CH_2$.

Ces résines sont des polymères d'organosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle $SiO_{4/2}$ dans laquelle l'atome de silicium est lié à quatre atomes d'hydrogène eux mêmes liés au reste du polymère. Comme exemples de telles résines, on peut citer les résines de silicone MT telles que les poly(phényl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend de préférence au moins deux groupes Si-H (groupes hydrogénosilanes) libres.

Le composé Y peut être avantageusement choisi parmi les polyorganosiloxanes comprenant au moins une unité alkylhydrogènosiloxane de formule suivante :

$$R_pHSiO_{\frac{(3-p)}{2}} \qquad \text{(III)}$$

dans laquelle :

R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y polyorganosiloxanes à unités alkylhydrogènosiloxanes peuvent comprendre en outre des unités de formule :

$$R_nSiO_{\frac{(4-n)}{2}} \qquad \text{(II)}$$

telles que définies plus haut.

Le composé Y peut être une résine de silicone comprenant au moins un motif choisi parmi les motifs M, D, T, Q tels que définis ci-dessus et comprenant au moins un groupe Si-H tel que les poly(méthyl-hydridosilsesquioxane) commercialisées sous la référence SST-3MH1.1 par la société Gelest.

De préférence, ces composés Y polyorganosiloxanes comprennent de 0,5 à 2,5% en poids de groupes Si-H.

Avantageusement, les radicaux R représentent un groupement méthyle dans les formules (I), (II), (III) ci-dessus.

De préférence, ces polyorganosiloxanes Y comprennent des groupes terminaux de formule $(CH_3)_3SiO_{1/2}$.

Avantageusement, les polyorganosiloxanes Y comprennent au moins deux unités alkylhydrogènosiloxane de formule $-(H_3C)(H)SiO-$ et comprennent éventuellement des unités $-(H_3C)_2SiO-$.

De tels composés polyorganosiloxanes Y à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé Y étant choisi parmi les polyorganosiloxanes Y à groupements hydrogénosilane cités ci-dessus.

Selon un mode de réalisation, les composés X organiques ou hybrides organique/silicone portant au moins 2 groupements aliphatiques insaturés réactifs, portent au moins un groupe polaire tel que décrit plus haut.

Le composé X, de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :

a) les polyesters à insaturation(s) éthylénique(s) :
Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
- d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.

b) les polyesters à groupes (méth)acrylate latéraux et/ou terminaux :
Il s'agit d'un groupe de polymères de type polyester obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).

c) les polyuréthannes et/ou polyurées à groupes (méth)acrylate, obtenus par polycondensation :

- de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule :

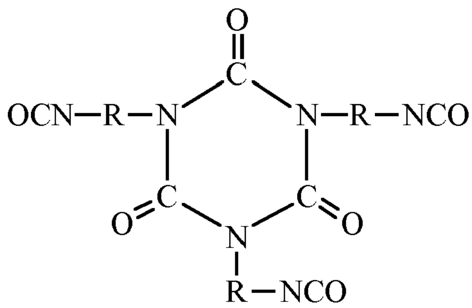

résultant de la trimérisation de 3 molécules de diisocyanates OCN-R-CNO, où R est un radical hydrocarboné linéaire, ramifié ou cyclique comportant de 2 à 30 atomes de carbone ;

- de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine

ou l'hexaméthylènediamine, et

- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

De tels polyuréthannes/polyurées à groupes acrylates sont commercialisés par exemple sous la dénomination SR 368 (tris(2-hydroxyéthyl)isocyanurate-triacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.

d) les polyéthers à groupes (méth)acrylate obtenus par estérification, par l'acide (méth)acrylique, des groupes hydroxyle terminaux d'homopolymères ou de copolymères d'alkylèneglycols en $C_{1-4}$, tels que le polyéthylèneglycol,

le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.
Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.

e) les époxyacrylates obtenus par réaction entre

- au moins un diépoxyde choisi par exemple parmi :

  (i) l'éther diglycidylique de bisphénol A,
  (ii) une résine diépoxy résultant de la réaction entre l'éther diglycidylique de bisphénol A et l'épichlorhydrine,
  (iii) une résine époxyester à extrémités $\alpha,\omega$-diépoxy résultant de la condensation d'un diacide carboxylique ayant de 3 à 50 atomes de carbone avec un excès stoechiométrique de (i) et/ou (ii),
  (iv) une résine époxyéther à extrémités $\alpha,\omega$-diépoxy résultant de la condensation d'un diol ayant de 3 à 50 atomes de carbone avec un excès stoechiométrique de (i) et/ou (ii),
  (v) les huiles naturelles ou synthétiques portant au moins 2 groupes époxyde, telles que l'huile de soja époxydée, l'huile de lin époxydée et l'huile de vernonia époxydée,
  (vi) un polycondensat phénol-formaldéhyde (résine Novolac®), dont les extrémités et/ou les groupes latéraux ont été époxydés,
  et

- un ou plusieurs acides carboxyliques ou polyacides carboxyliques comportant au moins une double liaison éthylénique en $\alpha,\beta$ du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.

De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.

f) les poly(méth)acrylates d'(alkyle en $C_{1-50}$), ledit alkyle étant linéaire, ramifié ou cyclique, comportant au moins deux fonctions à double liaison éthylénique portées par les chaînes hydrocarbonées latérales et/ou terminales.
De tels copolymères sont commercialisés par exemple sous les dénominations IRR® 375, OTA® 480 et EBECRYL® 2047 par la société UCB.

g) les polyoléfines telles que le polybutène, le polyisobutylène,

h) les perfluoropolyéthers à groupes acrylate obtenus par estérification, par exemple par l'acide (méth)acrylique, de perfluoropolyéthers portant des groupes hydroxyle latéraux et/ou terminaux.
De tels perfluoropolyéthers $\alpha,\omega$-diols sont décrits notamment dans EP-A-1057849 et sont commercialisés par la société AUSIMONT sous la dénomination FOMBLIN® Z DIOL.

i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.
Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.
Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754). La société PERSTORP commercialise sous la dénomination BOLTORN® des polyesters hyperramifiés. On trouvera sous la dénomination COMBURST® de la société DENDRITECH des polyéthylèneamines hyperramifiées. Des poly (esteramide) hyperramifiés à extrémités hydroxyle sont commercialisés par la société DSM sous la dénomination HYBRANE®.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

On peut donc utiliser ces polymères dendritiques et hyperramifiés en association avec un ou plusieurs des polymères et/ou oligomères a) à h) ci-dessus.

**1a - Composés réactifs additionnels**

Selon un mode de réalisation, les compositions comprenant le composé X et/ou Y peut comprendre en outre un composé réactif additionnel tels que :

- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetraméthyltetravinylsiloxane,
- des composés silazanes tels que l'hexaméthyldisilazane.

**1b - Catalyseur**

La réaction d'hydrosilylation se fait en présence d'un catalyseur qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur est de préférence à base de platine ou d'étain.

On peut citer par exemple les catalyseurs à base de platine déposé sur un support de gel de silice ou de poudre de charcoal (charbon), le chlorure de platine, les sels de platine et d'acides chloroplatiniques.

On utilise de préférence les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tetraméthyldisiloxane.

Le catalyseur peut être présent en une teneur allant de 0,0001% à 20% en poids par rapport au poids total de la composition le comprenant.

Les composés X et/ou Y peuvent être associés à des inhibiteurs ou retardateurs de polymérisation, et plus particulièrement des inhibiteurs du catalyseur. De façon non limitative, on peut citer les polyméthylvinylsiloxanes cycliques, et en particulier le tetravinyl tétraméthyl cyclotetrasiloxane, les alcools acétyléniques, de préférence volatils, tels que le méthylisobutynol.

La présence de sels ioniques, tels que l'acétate de sodium peut avoir une influence dans la vitesse de polymérisation des composés.

A titre d'exemple d'une combinaison de composés X et Y réagissant par hydrosilylation en présence d'un catalyseur, on peut citer les références suivantes proposée par la société Dow Corning : DC 7-9800 Soft Skin Adhesive Parts A & B, ainsi que la combinaison des mélanges A et B suivants préparés par Dow Corning :

**MELANGE A :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

De façon avantageuse, les composés X et Y sont choisis parmi les composés siliconés susceptibles de réagir par hydrosilylation en présence d'un catalyseur ; en particulier le composé X est choisi parmi les polyorganosiloxanes comprenant des unités de formule (I) décrits ci-dessus et le composé Y est choisi parmi les organosiloxanes comprenant des unités alkylhydrogènosiloxanes de formule (III) décrits ci-dessus.

Selon un mode de réalisation particulier, le composé X est un polydiméthylsiloxane à groupements vinyliques terminaux, et le composé Y est un polyméthylhydrogénosiloxane.

**2/ Composés X et Y susceptibles de réagir par condensation**

Selon un mode de réalisation, l'invention concerne un kit cosmétique utile pour le soin et/ou le maquillage de matière(s) kératinique(s), notamment de la peau, comprenant au moins deux compositions différentes et conditionnées séparément avec l'une au moins des compositions étant de type émulsion et comprenant au moins un polymère amphiphile, le kit comprenant un ou plusieurs composés X, un ou plusieurs composés Y, et éventuellement au moins un catalyseur, avec au moins un des composés X et Y étant siliconé et lesdits composés X et Y étant susceptibles de réagir ensemble par une réaction de condensation lorsqu'ils sont mis en contact les uns avec les autres, et dans lequel les composés X, Y et le catalyseur lorsqu'il est présent, ne sont pas présents simultanément dans la même composition.

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, celle-ci peut être en particulier l'humidité ambiante, l'eau résiduelle de la peau, des lèvres des cils et/ou des ongles, ou l'eau apportée par une source extérieure, par exemple par humidification préalable de la matière kératinique (par exemple par un brumisateur, des larmes naturelles ou artificielles).

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation, le composé X et/ou le composé Y est porteur d'au moins un groupe polaire, tel que décrit ci-dessus, susceptible de former au moins une liaison hydrogène avec les matières kératiniques.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et/ou Y comprennent de préférence de façon majoritaire des unités de formule :

$$R^9_sSiO_{(4-s)/2} \quad (IV)$$

dans laquelle les groupes $R^9$ représentent indépendamment les uns des autres un radical choisi parmi les groupes alkyle comprenant de 1 à 6 atomes de carbone, le phényle, les groupes fluoroalkyle, et s est égal à 0, 1, 2 ou 3. De préférence, les groupes $R^9$ représentent indépendamment les uns des autres un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule :

$$(R^9{}_2SiO_2)_f \quad (V)$$

dans laquelle $R^9$ est tel que décrit ci-dessus, de préférence $R^9$ est un radical méthyle, et f est tel que le polymère présente avantageusement une viscosité à 25°C allant de 0,5 à 3000 Pa.s, de préférence allant de 5 à 150 Pa.s ; par exemple f peut aller de 2 à 5000, de préférence de 3 à 3000, et préférentiellement de 5 à 1000.

Ces composés X et Y polyorganosiloxanes comprennent au moins 2 groupes trialcoxysilanes terminaux par molécule de polymère, lesdits groupes ayant la formule suivante

$$-ZSiR^1{}_x(OR)_{3-x} \quad (VI)$$

dans laquelle :

les radicaux R représentent indépendamment un groupe méthyle, éthyle, n-propyle, isopropyle, n-butyle, sec-butyle, isobutyle, de préférence un groupe méthyle ou éthyle,
$R^1$ est un groupe méthyle ou éthyle,
x est égal à 0 ou 1, de préférence x est égal à 0 et
Z est choisi parmi : les groupes hydrocarbonés divalents ne comportant pas d'insaturation éthylénique et comprenant de 1 à 18 atomes de carbone, de préférence de 2 à 18 atomes de carbone (groupes alkylène), les combinaisons de radicaux hydrocarbonés divalents et de segments siloxanes de formule (IX) suivante :

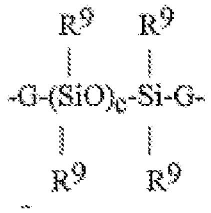

(IX)

$R^9$ étant tel que décrit plus haut, G est un radical hydrocarboné divalent ne comportant pas d'insaturation éthylénique et comprenant de 1 à 18 atomes de carbone, de préférence de 2 à 18 atomes de carbone et c est un entier allant de 1 à 6.
Z et G peuvent être notamment choisis parmi les groupements alkylènes tels que le méthylène, l'éthylène, le propylène, le butylène, le pentylène, l'hexylène, les groupements arylène tels que le phénylène.
De préférence, Z est un groupe alkylène, et mieux éthylène.
Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule $CH_2=CH-SiR^9{}_2-$ ou de formule $R^6{}_3-Si-$, dans laquelle $R^9$ est tel que défini plus haut et chaque groupe $R^6$ est indépendamment choisi parmi les groupes $R^9$ ou vinyle. On peut citer comme exemples de tels groupements terminaux les groupes triméthoxysilane, triéthoxysilane, vinyldiméthoxysilane et vinylméthylo xyphénylsilane.
De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.
On peut citer à titre de composé X et/ou Y en particulier les polyorganosiloxanes choisis parmi les polymères de formule :

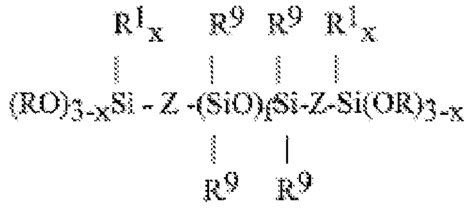

(VII)

dans laquelle R, $R^1$, $R^9$, Z, x et f sont tels que décrits plus haut.

Les composés X et/ou Y peuvent également comprendre un mélanges de polymère de formule (VII) ci-dessus avec des polymères de formule (VIII) suivante :

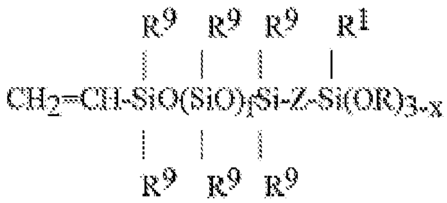

(VIII)

dans laquelle R, $R^1$, $R^9$, Z, x et f sont tels que décrits plus haut.

Lorsque le composé X et/ou Y polyorganosiloxanes à groupe(s) alcoxysilane(s) comprend un tel mélange, les différents polyorganosiloxanes sont présents en des teneurs telles que les chaînes organosilyles terminales représentent moins de 40%, de préférence moins de 25% en nombre des chaînes terminales.

Les composés X et/ou Y polyorganosiloxanes particulièrement préférés sont ceux de formule (VII) décrits ci-dessus. De tels composés X et/ou Y sont décrits par exemple dans le document WO 01/96450.

Comme indiqué plus haut, les composés X et Y peuvent être identiques ou différents.

En particulier, les composés X et Y peuvent représenter un mélange de polydiméthylsiloxanes à groupements méthoxysilanes.

Selon une variante, l'un des 2 composés réactifs X ou Y, est de nature siliconée et l'autre est de nature organique. Par exemple le composé X est choisi parmi les oligomères ou polymères organiques ou les oligomères ou polymères hybrides organique/silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Selon un mode de réalisation, le composé X de nature organique ou de nature hybride organique/silicone est porteur d'au moins un groupe polaire, tel que décrit ci-dessus, susceptible de former au moins une liaison hydrogène avec la matière kératinique.

Les polymères organiques de nature vinylique ou (méth)acryliques, porteurs de groupes latéraux alcoxysilanes, pourront en particulier être obtenus par copolymérisation d'au moins un monomère organique vinylique ou (méth) acrylique avec un (méth)acryloxypropyltriméthoxysilane, un vinyl triméthoxysilane, un vinyltriéthoxysilane, un allyltriméthoxysilanes etc..

On peut citer par exemple les polymère (méth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et/ou polyurées, polyéthers, et porteurs de groupes alcoysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane :

aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux, on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11 , pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.
A titre de composés X et/ou Y polyorganosiloxane, on peut citer les résines de type MQ ou MT portant elle-même des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

2a - Composé réactif additionnel

Selon un mode de réalisation, le composé X et/ou Y peut être associé en outre à un composé réactif additionnel comprenant au moins deux groupes alcoxysilane ou silanol.

On peut citer par exemple :

- une ou des particules organiques ou minérales comprenant à leur surface des groupements alcoxysilanes et/ou silanols, par exemple des charges traitées en surface par de tels groupes.

2b - Catalyseur

La réaction de condensation peut se faire en présence d'un catalyseur à base de métal qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur utile dans ce type de réaction est de préférence un catalyseur à base de titane.

On peut citer notamment les catalyseurs à base de tetraalcoxytitane de formule :
$Ti(OR^2)_y(OR^3)_{4-y}$,
dans laquelle $R^2$ est choisi parmi les radicaux alkyle tertiaires tels que le tert butyle, le tert amyle et le 2,4-diméthyl-3-pentyle ; $R^3$ représente un radical alkyle comprenant de 1 à 6 atomes de carbone, de préférence un groupe méthyle, éthyle, n-propyle, isopropyle, n-butyle, sec-butyle, hexyle et y est un nombre allant de 3 à 4, mieux de 3,4 à 4.

Le catalyseur peut être présent en une teneur allant de 0,0001% à 20% en poids par rapport au poids total de la composition le contenant.

**2c - Diluant**

Les compositions utiles comprenant X et/ou Y peuvent comprendre en outre une huile siliconée volatile (ou diluant) destinée à faire diminuer la viscosité de la composition. Cette huile peut être choisie parmi les silicones linéaires à chaîne courte telles que l'hexaméthyldisiloxane, l'octaméthyltrisiloxane, les silicones cycliques telles que l'octaméthylcyclotetrasiloxane, la decaméthylclopentasiloxane et leurs mélanges.

Cette huile siliconée peut représenter de 5% à 95%, de préférence de 10 à 80% en poids par rapport au poids de chaque composition.

A titre d'exemple d'une combinaison de composés X et Y porteurs de groupements alcoxysilanes et réagissant par condensation, on peut citer la combinaison des mélanges A' et B' suivants préparés par la société Dow Corning :

**Mélange A' :**

| **Ingrédient (Nom INCI)** | **N°CAS** | **Teneurs (%)** | **Fonction** |
|---|---|---|---|
| Bis-Trimethoxysiloxyethyl Tetramethyldisilo xyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

**Mélange B' :**

| **Ingrédient (Nom INCI)** | **N°CAS** | **Teneurs (%)** | **Fonction** |
|---|---|---|---|
| Disiloxane | 107-46-0 | 80-99 | Solvant |
| Tetra T Butyl Titanate | - | 1-20 | Catalyseur |

Il est à noter que les composés X et Y, identiques, sont réunis dans le mélange A' (cf. (1))

**3/ Réticulation en présence de peroxyde :**

Selon un mode de réalisation, l'invention concerne un kit cosmétique utile pour le soin et/ou le maquillage de matière(s) kératinique(s), notamment de la peau, comprenant au moins deux compositions différentes et conditionnées séparément avec l'une au moins des compositions étant de type émulsion et comprenant au moins un polymère amphiphile, le kit comprenant un ou plusieurs composés X, un ou plusieurs composés Y, et au moins un peroxyde, avec au moins un des composés X et Y étant siliconé et lesdits composés X et Y étant susceptibles de réagir ensemble par une réaction de réticulation en présence d'un peroxyde lorsqu'ils sont mis en contact les uns avec les autres, et dans lequel les composés X, Y et le peroxyde ne sont pas présents simultanément dans la même composition.
Cette réaction se fait de préférence par chauffage à une température supérieure ou égale à 50°C, de préférence supérieure ou égale à 80°C, allant jusqu'à 120°C.
Les composés X et Y, identiques ou différents, comprennent dans ce cas au moins deux groupements latéraux $-CH_3$ et/ou au moins deux chaînes latérales portant un groupement $-CH_3$.
Les composés X et Y sont de préférence siliconés et peuvent être choisis par exemple parmi les polydiméthylsiloxanes linéaires non volatiles de haut poids moléculaire, ayant un degré de polymérisation supérieur à 6 présentant au moins deux groupements latéraux $-CH_3$ reliés à l'atome de silicium et/ou au moins deux chaînes latérales portant un groupement $-CH_3$. On peut citer par exemple les polymères décrits dans le Catalogue « Reactive Silicones » de la société Gelest Inc., Edition 2004, page 6, et notamment les copolymères (aussi appelés gommes) de vinylméthylsiloxane-diméthylsiloxane de poids moléculaires allant de 500 000 à 900 000 et notamment de viscosité supérieure à 2 000 000 cSt.
A titre de peroxydes utilisables dans le cadre de l'invention, on peut citer le peroxyde de benzoyle, le peroxyde de 2,4-dichlorobenzoyle et leurs mélanges.
Selon un mode de réalisation, la réaction d'hydrosilylation en présence d'un catalyseur, ou la réaction de condensation, ou bien encore la réaction de réticulation en présence d'un peroxyde, entre les composés X et Y est accélérée par un apport de chaleur en élevant par exemple la température du système entre 25°C et 180°C.
D'une façon générale, quel que soit le type de réaction par laquelle les composés X et Y réagissent ensemble, le pourcentage molaire de X par rapport à l'ensemble des composés X et Y, c'est-à-dire le ratio X/(X+Y) x 100, peut varier de 5% à 95%, de préférence de 10% à 90%, mieux encore de 20% à 80%.
De même, le pourcentage molaire de Y par rapport à l'ensemble des composés X et Y, c'est-à-dire le ratio Y/(X+Y) x 100, peut varier de 5% à 95%, de préférence de 10% à 90%, mieux encore de 20% à 80%.
Le composé X peut présenter une masse moléculaire moyenne en poids (Mw) allant de 150 à 1 000 000, de préférence de 200 à 800 000, de préférence encore de 200 à 250 000.
Le composé Y peut présenter une masse moléculaire moyenne en poids (Mw) allant de 200 à 1 000 000, de préférence de 300 à 800 000, de préférence encore de 500 à 250 000.
Le composé X peut représenter de 0,1 % à 95% en poids par rapport au poids total de la composition le contenant, de préférence de 1% à 90%, et mieux de 5% à 80%.
Le composé Y peut représenter de 0.1 % à 95% en poids par rapport au poids total de la composition le contenant, de préférence de 1% à 90% et mieux de 5% à 80%.
Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.
En particulier, les composés X et Y peuvent être présents en un ratio X/Y molaire allant de 0,05 à 20 et mieux de 0,1 à 10.
Les composés X et Y peuvent avantageusement être associés à au moins une charge. Ainsi, le kit selon l'invention peut par exemple comprendre dans au moins une des compositions une charge choisie parmi la silice ou la silice traitée en surface.
Comme précisé précédemment, selon un mode de réalisation de l'invention, les composés X et Y peuvent être mis en oeuvre sous la forme d'une unique composition qui contient alors au moins l'un d'entre eux ou le cas échéant le catalyseur ou le peroxyde si nécessaire à leur interaction, sous une forme encapsulée.

Dans le cadre de la présente invention, sont plus particulièrement considérées les formes encapsulées de type coeur/écorce dites encore microcapsules ou nanocapsules dont l'écorce est de nature polymérique et le coeur contient le composé X, le composé Y, l'un de ses composés X et Y étant le cas échéant encapsulé avec le catalyseur ou le peroxyde si nécessaire à l'interaction des deux composés. Dans l'hypothèse où ce catalyseur n'est pas encapsulé avec l'un ou l'autre des composés X ou Y, il est présent dans la composition cosmétique contenant les formes encapsulées.
De nombreuses techniques sont à ce jour disponibles pour préparer ce type de microcapsules ou nanocapsules
Toutefois, selon un mode préféré, les formes encapsulées considérées selon l'invention sont des nanocapsules et sont obtenues par une technique dite basculement de solvant notamment illustrée dans les documents EP 274 961 et EP 1 552 820.
Plus particulièrement, l'écorce des nanocapsules de composé X ou Y, mises en oeuvre selon l'invention, est de nature polymérique, non réticulée, non hydrosoluble et non soluble dans le coeur des capsules.
D'une manière générale, tous les polymères, d'origine naturelle ou synthétique, solubles dans un solvant non miscible à l'eau et notamment ceux ayant un point de fusion inférieur au point d'ébullition de l'eau à la pression atmosphérique (100 °C), peuvent convenir.
Ces polymères peuvent être biodégradables, comme par exemple les polyesters, ou non.
A titre illustratif des polymères convenant à l'invention, on peut notamment citer :

- les polymères de cyanoacrylate d'alkyle en $C_2$-$C_{12}$
- les polymères formés par les poly-L-lactides, les poly-DL-lactides, les polyglycolides et les copolymères correspondants,
- les polycaprolactones,
- les polymères de l'acide 3-hydroxy butyrique,
- les copolymères de chlorure de vinyle et d'acétate de vinyle,
- les copolymères d'acide et d'ester méthacrylique, notamment d'acide méthacrylique et d'ester d'acide méthacrylique,
- l'acéto-phtalate de polyvinyle,
- l'acéto-phtalate de cellulose,
- le copolymère polyvinylpyrrolidone-acétate de vinyle,
- les polyéthylènevinylacétates,
- les polyacrylonitriles,
- les polyacrylamides,
- les polyéthylèneglycols,
- les poly-(méthacrylate d'hydroxyalkyle en $C_1$ à $C_4$)
- les esters de cellulose et d'acide carboxylique en $C_1$-$C_4$ ;,
- le polystyrène et les copolymères de styrène et d'anhydride maléique, les copolymères de styrène et d'acide acrylique, les terpolymères séquencés styrène éthylène/butylène-styrène, les terpolymères séquencés styrène-éthylène/propylène-styrène,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes dont les polydiméthylsiloxanes,
- les poly (alkylène adipate),
- les polyesters polyol,
- les polymères siliconés de polysilsesquioxane, ,
- les polyesters dendritiques à fonction hydroxyle terminale,
- les polymères hydrodipersibles mais néanmoins solubles dans des solvants non miscibles à l'eau comme par exemple : les polyesters, poly(ester amides), polyuréthannes et copolymères vinyliques portant des fonctions acide carboxylique et/ou sulfonique et en particulier ceux décrits dans le document FR 2 787 729,
- les copolymères blocs insolubles dans l'eau à température ambiante et solides à température ambiante, ayant au moins un bloc d'un des polymères précédents, et
- leurs mélanges.

Ces polymères ou copolymères peuvent posséder un poids moléculaire moyen en poids compris entre 1000 et 500 000 et en particulier entre 1500 et 100 000.
Conviennent tout particulièrement à l'invention, les poly(alkylène adipate), les organo polysiloxanes, les polycaprolactones, l'acétophtalate de cellulose, l'acétobutyrate de cellulose, les esters de cellulose, le polystyrène, et ses dérivés, et notamment les polycaprolactones.

Bien entendu, l'homme du métier est à même, de par ses connaissances, d'ajuster le poids moléculaire du polymère sélectionné vis-à-vis de sa concentration dans le solvant afin d'avoir une viscosité du mélange compatible avec une émulsification satisfaisante.
En ce qui concerne le coeur lipophile, il peut contenir outre le composé X ou le composé Y, au moins une huile. L'huile peut être choisie parmi les huiles décrites ci-après pour la phase huileuse. L'huile est de préférence une huile siliconée.
Selon une variante de l'invention les formes encapsulées de composé X ou composé Y peuvent être enrobées d'une phase lamellaire.
En ce qui concerne le protocole opératoire pour préparer des nanocapsules convenant à l'invention, l'homme de l'art pourra se reporter notamment à l'enseignement du document EP 1 552 820 cité précédemment. Le choix des tensioactifs requis ainsi que la mise en oeuvre du procédé fait appel aux connaissances de l'homme de l'art.

## MILIEU PHYSIOLOGIQUEMENT ACCEPTABLE

Les compositions selon l'invention peuvent comprendre un milieu physiologiquement acceptable, c'est à dire un milieu non toxique et susceptible d'être appliqué sur les matières kératiniques d'êtres humains et d'aspect, d'odeur et de toucher agréables.
Lorsque les compositions selon l'invention sont sous la forme d'une émulsion, celle-ci peut être de type émulsions simples obtenues par dispersion d'une phase grasse dans une phase aqueuse ou inversement ou de type émulsion multiple. Plus particulièrement il s'agit d'une émulsion simple. Ces compositions sont préparées selon les méthodes usuelles.
Ainsi, les compositions selon l'invention peuvent avantageusement se présenter sous forme d'une émulsion obtenue par dispersion d'une phase aqueuse dans une phase grasse (E/H) ou d'une phase grasse dans une phase aqueuse (H/E), de consistance liquide ou semi-liquide du type lait, ou de consistance molle, semi-solide ou solide du type crème ou gel, ou encore d'émulsion multiple (E/H/E ou H/E/H). Ces compositions sont préparées selon les méthodes usuelles.

### Définition phase huileuse :

L'une au moins des première et seconde compositions peut comprendre une phase grasse liquide.
La phase grasse liquide peut comprendre au moins une huile et en particulier une huile choisie parmi les huiles hydrocarbonées et/ou siliconés et/ou fluorées.
Comme exemples d'huiles utilisables dans la composition selon l'invention, on peut citer :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R_1COOR_2$ et $R_1OR_2$ dans laquelle $R_1$ représente le reste d'un acide gras comportant de 8 à 29 atomes de carbone, et $R_2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam® ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool

oléique ou l'alcool linoléique ;

- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; Ces corps gras peuvent être choisis de manière variée par l'homme du métier afin de préparer des compositions ayant les propriétés, par exemple de consistance ou de texture, souhaitées. En particulier ils se doivent d'être compatibles avec la formulation des compositions sous la forme d'une d'émulsion.

Les compositions selon l'invention peuvent en outre comprendre une huile volatile. Par " huile volatile", on entend au sens de l'invention une huile susceptible de s'évaporer au contact des matières kératiniques en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa (10-3 à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).

On peut citer comme huiles volatiles entre autres, les silicones cycliques ou linéaires renfermant de 2 à 6 atomes de silicium, telles que le cyclohexasiloxane, le dodecamethylpentasiloxane, le decamethyltetrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de "PF 5050®" et "PF 5060®" par la Société 3M et les dérivés de perfluoromorpholine, tels que la 4-trifluorométhyl perfluoromorpholine vendue sous la dénomination "PF 5052®" par la Société 3M.

Les première et seconde compositions peuvent être exemptes d'huile volatile.

La ou les huiles peuvent être présentes dans les compositions selon l'invention, en particulier lorsque la composition est une émulsion à phase continue aqueuse, en une teneur allant de 2 à 60 % en poids, de préférence de 2 à 50 % en poids par rapport au poins total de la composition. Comme précisé précédemment, les compositions de type émulsion contiennent au moins à titre d'agent émulsionnant un polymère amphiphile.

**Polymère amphiphile**

Le polymère amphiphile selon l'invention peut être un polymère hydrosoluble ou hydrodispersible amphiphile ou un polymère liposoluble ou lipodispersible amphiphile. En particulier, le polymère est hydrosoluble ou hydrodispersible, notamment lorsque la composition est une émulsion à phase continue aqueuse.

Par « polymère hydrosoluble ou hydrodispersible amphiphile », on entend un polymère qui, introduit en solution aqueuse à 0,05% (en poids), permet de réduire la tension superficielle de l'eau à 25°C à une valeur inférieure à 50 mN/m, et de préférence inférieure à 40 mN/m.

Par « polymère liposoluble ou lipodispersible amphiphile », on entend un polymère qui, introduit dans une huile à 0,05 % (en poids), permet de réduire sa tension superficielle à 25 °C d'au moins 5 mN/m, et de préférence d'au moins 10 mN/m.

Le rapport pondéral huile(s) sur polymère, en particulier lorsque la composition est une émulsion à phase continue aqueuse, est de préférence compris entre 2 et 80 voire entre 5 et 50.

*1) Polymère hydrosoluble ou hydrodispersible amphiphile*

Par « polymère hydrosoluble ou hydrodispersible », on entend un polymère qui, introduit dans de l'eau à une

concentration égale à 1 %, conduit à une solution macroscopiquement homogène dont la transmittance de la lumière, à une longueur d'onde égale à 500 nm, à travers un échantillon de 1 cm d'épaisseur, est d'au moins 10 %, ce qui correspond à une absorbance [abs = -log(transmittance)] inférieure à 1,5.

Les polymères de l'invention peuvent être des polymères à blocs, ou des polymères greffés, qui comprennent d'une part au moins une séquence polymérique hydrosoluble ou hydrodispersible et d'autre part au moins une séquence hydrophobe.

Les polymères employés dans le cadre de l'invention peuvent donc être des polymères à blocs, comprenant par exemple des séquences hydrosolubles alternées avec des séquences hydrophobes.

Ces polymères peuvent également se présenter sous la forme de polymères greffés dont le squelette est hydrosoluble ou hydrodispersible, et porte des greffons hydrophobes. Cette structure peut être partiellement réticulée.

*- Les séquences polymériques hydrosolubles*

Par « séquences hydrosolubles », on entend des séquences qui, introduites dans de l'eau à une concentration égale à 1 %, conduisent à une solution macroscopiquement homogène dont la transmittance de la lumière, à une longueur d'onde égale à 500 nm, à travers un échantillon de 1 cm d'épaisseur, est d'au moins 10 %, ce qui correspond à une absorbance [abs = - log(transmittance)] inférieure à 1,5.

Ces séquences hydrosolubles peuvent être obtenues par polymérisation radicalaire de monomères vinyliques, ou par polycondensation, ou encore peuvent être constituées par des polymères naturels ou naturels modifiés existants.

A titre d'exemple, on peut citer les monomères hydrosolubles suivants et leurs sels, qui sont susceptibles d'être employés pour former lesdites unités hydrosolubles ou hydrodispersibles, seuls ou en mélange :

- l'acide (méth)acrylique,
- l'acide vinylsulfonique et l'acide (meth)allylsulfonique,
- l'acide vinylphosphonique,
- le chlorure de méthyl vinylimidazolium,
- le (méth)acrylamide,
- la 2-vinylpyridine et la 4-vinylpyridine,
- l'acide et l'anhydride maléiques;
- l'acide crotonique,
- l'acide itaconique ;
- l'alcool vinylique de formule $CH_2$=CHOH;
- les N-vinyllactames tels que la N-vinylpyrrolidone, la N-vinylcaprolactame et la N-butyrolactame;
- les dérivés hydrosolubles du styrène, notamment le styrène sulfonate;
- le chlorure de diméthyldiallyl ammonium;
- la N-vinylacétamide et la N-méthyl N-vinylacétamide,
- la N-vinylformamide et la N-méthylvinylformamide,
- les monomères vinyliques hydrosolubles de formule (1) suivante :

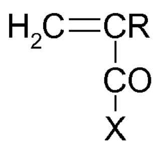

(1)

dans laquelle :

- R est choisi parmi H, $-CH_3$, $-C_2H_5$ ou $-C_3H_7$
- X est choisi parmi :

    - les oxydes d'alkyle de type -OR' où R' est un radical hydrocarboné, linéaire ou ramifié, saturé ou insaturé, ayant de 1 à 6 carbones, substitué par au moins un atome d'halogène (iode, brome, chlore, fluor); un groupement sulfonique ($-SO_3^-$), sulfate ($-SO_4^-$), phosphate ($-PO_4H_2^-$); hydroxy (-OH); éther (-O-) ; amine primaire ($-NH_2$); amine secondaire ($-NHR_1$), tertiaire ($-NR_1R_2$) ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 6 atomes de carbone, sous réserve que la somme des atomes de carbone de R' + $R_1$ + $R_2$ + $R_3$ ne dépasse pas 7 ; Citons le méthacrylate de diméthylaminoéthyl quaternisé (MADAME), le (méth)acrylate de glycidyle, le méthacrylate

d'hydroxyéthyle, et les (méth)acrylates d'éthylène glycol, de diéthylèneglycol ou de polyalkylèneglycol,

- les groupements $-NH_2$, -NHR' et -NR'R" dans lesquels R' et R" sont indépendamment l'un de l'autre des radicaux hydrocarbonés, linéaires ou ramifiés, saturés

ou insaturés ayant 1 à 6 atomes de carbone, sous réserve que le nombre total d'atomes de carbone de R' + R" ne dépasse pas 7, lesdits R' et/ou R" étant substitués par un atome d'halogène (iode, brome, chlore, fluor) ; un groupement hydroxy (-OH); éther (-O-), sulfonique ($-SO_3^-$); sulfate ($-SO_4^-$); phosphate ($-PO_4H_2$); amine primaire ($-NH_2$); amine secondaire ($-NHR_1$), tertiaire ($-NR_1R_2$) et/ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 6 atomes de carbone, sous réserve que la somme des atomes de carbone de R' + R" + $R_1$ + $R_2$ + $R_3$ ne dépasse pas 7 ; Citons la N,N diméthylacrylamide, l'acide acrylamido-2-méthylpropane sulfonique (AMPS), le chlorure de (méth)acrylamido propyl triméthyl ammonium (APTAC et MAPTAC).

Les séquences hydrosolubles peuvent également être obtenu à partir de monomères hydrophobes, les dits monomères hydrophobes étant présents en une quantité suffisamment faible pour que ces unités soient hydrosolubles. Citons par exemple comme monomères hydrophobes :

- le styrène et ses dérivés tel que le 4-butylstyrène, l'alpha méthylstyrène et le vinyltoluène,
- l'acétate de vinyle de formule $CH_2$=CH-$OCOCH_3$ ;
- les vinyléthers de formule $CH_2$=CHOR dans laquelle R est un radical hydrocarboné, linéaire ou ramifié, saturé ou insaturé, ayant de 1 à 6 carbones;
- l'acrylonitrile,
- la caprolactone,
- le chlorure de vinyle et le chlorure de vinylidène,
- les dérivés siliconés tels que le méthacryloxypropyltris(triméthylsiloxy)silane et les méthacrylamides siliconés,
- les monomères vinyliques hydrophobes de formule (2) suivante :

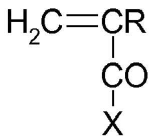

(2)

dans laquelle :

- R est choisi parmi H, $-CH_3$, $-C_2H_5$ ou $-C_3H_7$
- X est choisi parmi :
  - les oxydes d'alkyle de type -OR' où R' est un radical hydrocarboné, linéaire ou ramifié, saturé ou insaturé, ayant de 1 à 6 atomes de carbone.
  - les groupements $-NH_2$, -NHR' et -NR'R" dans lesquels R' et R" sont indépendamment l'un de l'autre des radicaux hydrocarbonés, linéaires ou ramifiés, saturés

ou insaturés ayant 1 à 6 atomes de carbone, sous réserve que le nombre total d'atomes de carbone de R' + R" ne dépasse pas 6. Citons par exemple, le méthacrylate de méthyle, le méthacrylate d'éthyle, le (meth)acrylate de n-butyle, le (meth)acrylate de tertio-butyle, l'acrylate de cyclohexyle et l'acrylate d'isobornyle et l'acrylate d'éthyle 2-hexyle.

Les séquences hydrosolubles peuvent être non neutralisées ou bien neutralisées totalement ou partiellement par une base minérale ou organique. Cette base peut être choisie, par exemple, parmi les sels de sodium, ammonium, lithium, calcium, magnésium, ammonium substitué par 1 à 4 groupes alkyle portant de 1 à 15 atomes de carbone, ou encore parmi la mono-, la di-, et la triéthanolamine, l'aminoéthylpropanediol, la N-méthyl-glucamine, et les acides aminés basiques, tels que l'arginine et la lysine, et leurs mélanges.

Le squelette des polymères greffés peut être réticulé ou non-réticulé ; Il est de préférence non-réticulé.

Les agents de réticulation peuvent être choisis parmi les composés à polyinsaturation oléfinique couramment utilisés pour la réticulation des polymères obtenus par polymérisation radicalaires. On peut citer par exemple le divinylbenzène, l'éther diallylique, le dipropylèneglycol-diallyléther, les polyglycol-diallyléthers, le triéthylèneglycol-divinyléther,

l'hydroquinone-diallyl-éther, le tétrallyl-oxéthanoyle ou d'autres allyl- ou vinyléthers alcools polyfonctionnels, le diacrylate de tétraéthylèneglycol, la triallylamine, le triméthylolpropane-diallyléther, composé à polyinsaturation oléfinique choisi parmi le divinylbenzène, le tétraallyloxyéthane, le méthylène-bis-acrylamide, les éthers allyliques d'alcools de la série des sucres, le méthylène-bis-acrylamide, le méthacrylate d'allyle, le triméthylol propane triacrylate (TMPTA) ou leurs mélanges.

Parmi les polycondensats et les polymères naturels ou naturels modifiés susceptibles de constituer tout ou partie des séquences hydrosolubles, on peut citer :

- les polyuréthanes hydrosolubles,
- la polyéthylèneimine,
- les polyéthers tels que le polyoxyéthylène et le polyoxypropylène,
- la gomme xanthane, notamment celle commercialisée sous les dénominations Keltrol T® et Keltrol SF® par Kelco; ou Rhodigel SM® et Rhodigel 200® de Rhodia ;
- les alginates (Kelcosol® de Monsanto) et leurs dérivés tels que l'alginate de propylène glycol (Kelcoloid LVF® de Kelco) ;
- les carraghenanes de type iota, kappa et lambda,
- les pectines de différents degrés de modification (HM et LM),
- les dérivés de cellulose et notamment la carboxyméthylcellulose (Aquasorb A500®, Hercules), l'hydroxyméthylcellulose, l'hydroxypropylcellulose, l'hydroxypropylméthylcellulose, l'hydroxyéthylcellulose et l'hydroxyéthylcellulose quaternisée ;
- les galactomannanes et leurs dérivés, tels que la gomme Konjac, Gellane, Caroube, Fennugrec, Karaya, Tragacanth, arabique, acacia, la gomme de guar, l'hydroxypropylguar, l'hydroxypropylguar modifié par des groupements méthylcarboxylate de sodium (Jaguar XC97-1®, Rhodia), le chlorure de guar hydroxypropyl tri-méthyl ammonium.
- les dérivés de l'amidon.

  Les séquences polymériques hydrosolubles ont une masse molaire moyenne comprise entre 1000 g/mole et 10 000 000 g/mole lorsqu'elles constituent le squelette hydrosoluble d'un polymère greffé.

  Ces séquences hydrosolubles ont de préférence une masse molaire comprise entre 500 g/mole et 500 000 g/mole lorsqu'elles constituent un bloc d'un polymère multiblocs.

*- Les séquences hydrophobes*

Par « séquences hydrophobes », on entend des séquences solubles ou dispersibles dans les corps gras que sont les alcanes, esters, éthers, triglycérides et/ou silicones, fluorées ou un mélange des huiles précédemment citées.

Ces séquences, introduites dans une huile ou un mélange huileux, à une concentration égale à 1 %, sous agitation à 50 °C pendant 48 heures, conduisent après retour à la température ambiante à une solution macroscopiquement homogène dont la transmittance de la lumière, à une longueur d'onde égale à 500 nm, à travers un échantillon de 1 cm d'épaisseur, est d'au moins 10 %, de préférence d'au moins 20 %. L'huile considérée peut être de type alcane, ester, triglycéride, éther, siliconée, fluorée ou un mélange des huiles précédemment citées.

Citons par exemples les séquences hydrophobes comprenant :

- un radical alkyle comportant de 6 à 30 atomes de carbones,
- un radical fluoré ou alkylfluoré en $C_6$-$C_{30}$ (par exemple le groupement de formule $-(CH_2)_2-(CF_2)_9-CF_3)$),
- un radical cholestéryle ou un radical dérivé de cholestérol (par exemple l'hexanoate de cholestéryle),
- un ou plusieurs groupe(s) cyclique(s) aromatique(s) comme le benzène, le naphtalène ou le pyrène,
- un radical siliconé ou alkylsiliconé ou encore alkylfluorosiliconé.

La masse molaire de ces séquences hydrophobes est comprise entre 100 et 10 000 g/mole, de préférence comprise entre 200 et 5000 g/mole.

La proportion massique des séquences hydrophobes dans le polymère final est de préférence comprise entre 1 % et 60 %, notamment entre 2 % et 40 %, et particulièrement entre 5 % et 30 % en poids, par rapport au polymère final.

*La synthèse*

Les polymères employés dans le cadre de l'invention peuvent être aisément préparés suivant différentes méthodes. Les polymères greffés peuvent ainsi être préparé par copolymérisation. Cette méthode consiste à copolymériser par exemple un macromonomère comportant une séquence hydrophobe (séquence hydrophobe précédemment décrite avec une extrémité vinylique) et un monomère vinylique hydrosoluble tel que l'acide acrylique ou les monomères vinyliques ayant la formule (1).

Les polymères blocs peuvent être préparés par réaction de couplage, par polymérisation vivante ou par polymérisation radicalaire contrôlée. Ainsi lorsque le polymère final se présente sous la forme d'un polymère à blocs, il est possible de le préparer par réaction de couplage entre des séquences hydrosolubles et des séquences hydrophobes ayant à chaque extrémité des sites réactifs complémentaires.

Il est également possible de préparer les polymères de l'invention par polymérisation vivante de type anionique ou cationique, ou bien par polymérisation radicalaire contrôlée. Ce dernier procédé de synthèse peut être mis en oeuvre suivant différents procédés comme par exemple la voie par transfert d'atomes (Atom Transfert Radical Polymerization ou ATRP), la méthode des radicaux tels que les nitroxydes ou encore la voie par transfert de chaîne réversible avec addition-fragmentation (Radical Addition-Fragmentation chain Transfert) telle que le procédé MADIX (Macromolecular Design via the Interchange of Xanthate). Ces procédés de synthèse peuvent être utilisés pour obtenir les blocs hydrosolubles et les blocs hydrophobes des polymères de l'invention ; ils peuvent également être utilisés pour synthétiser un seul des deux types de blocs du polymère de l'invention, l'autre bloc étant introduit dans le polymère final par l'intermédiaire de l'amorceur utilisé ou bien par réaction de couplage entre les blocs hydrosolubles et hydrophobes.

Le polymère hydrosoluble ou hydrodispersible amphiphile peut être choisi parmi les polymères dérivés d'acide acrylamido-2-méthylpropane sulfonique (AMPS), les polymères dérivés d'acide acrylique, les dérivés de polyéthers, les polymères amphiphiles issus des polymères naturels et leurs mélanges.

Les polymères conformes à l'invention, notamment les polymères dérivés d'AMPS et les polymères dérivés d'acide acrylique, ont en général une masse molaire moyenne en poids allant de 50 000 à 10 000 000, plus préférentiellement de 100 000 à 8 000 000 et encore plus préférentiellement de 200 000 à 3 000 000.

Les polymères conformes à l'invention, notamment les polymères dérivés d'AMPS et les polymères dérivés d'acide acrylique, de façon préférentielle sont neutralisés partiellement ou totalement par une base minérale (par exemple soude, potasse, ammoniaque) ou une base organique telle que la mono-, di- et tri-éthanolamine, une aminométhylpropanediol, la N- méthyl-glucamine, les acides aminés basiques comme l'arginine et la lysine, et leurs mélanges.

Les polymères amphiphiles, notamment les polymères dérivés d'AMPS et les polymères dérivés d'acide acrylique, selon l'invention peuvent être réticulés ou non-réticulés ; de préférence, ils sont non réticulés.

a) <u>les polymères dérivés d'AMPS (AcrylamidoMéthylPropane Sulfonique)</u>

Le polymère hydrosoluble ou hydrodispersible amphiphile selon l'invention est notamment un polymère dérivé d'AMPS. Les polymères dérivés d'AMPS comprennent :

- de 80 à 99 % en moles de motif acide 2-acrylamido 2-méthylpropane sulfonique (AMPS) de formule (3) suivante :

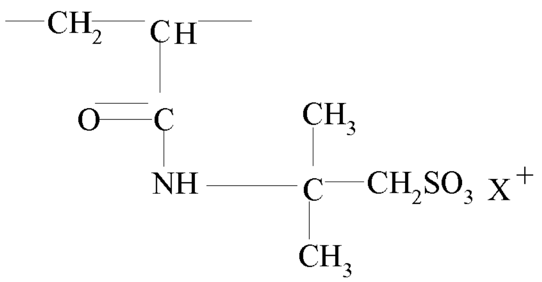

(3)

dans laquelle $X^+$ est un proton, un cation de métal alcalin, un cation alcalino-terreux ou l'ion ammonium ; et

- de 1 à 20 % en moles, et de préférence de 1 à 15 % en moles de motif de formule (4) suivante :

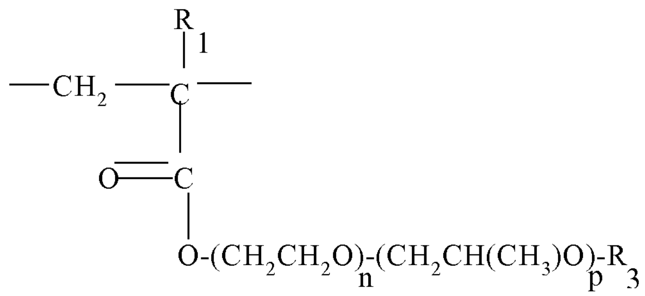

(4)

dans laquelle n et p, indépendamment l'un de l'autre désignent un nombre de moles et varie de 0 à 30, de préférence de 1 à 20 sous réserve que n + p soit inférieur ou égal à 30, de préférence inférieur à 25 et encore mieux inférieur à 20 ; $R_1$ désigne un atome d'hydrogène, un radical alkyle linéaire ou ramifié en $C_1$-$C_6$ (de préférence méthyle) et $R_3$ désigne un alkyle linéaire ou ramifié comportant m atomes de carbone, avec m allant de 6 à 30, de préférence de 10 à 25.

Les polymères amphiphiles utilisés selon l'invention peuvent être obtenus selon les procédés classiques de polymérisation radicalaire en présence d'un ou plusieurs initiateurs tels que par exemple l'azobisisobutyronitrile (AIBN), l'azobisdiméthylvaléronitrile, le chlorhydrate de 2,2-azobis-[2-amidinopropane](ABAH = 2,2-azoBis-[2-Amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou $H_20_2$ éventuellement en présence de réducteurs.

Les polymères sont notamment obtenus par polymérisation radicalaire en milieu tert-butanol dans lequel ils précipitent. En utilisant la polymérisation dans le tert-butanol, il est possible d'obtenir une distribution de la taille des particules de polymère particulièrement favorable pour ses utilisations.

La réaction de polymérisation peut être conduite à une température comprise entre 0 °C et 150 °C, de préférence entre 20 °C et 100 °C, soit à pression atmosphérique, soit sous pression réduite. Elle peut aussi être réalisée sous atmosphère inerte, et de préférence sous azote.

Selon ce procédé, on peut obtenir le polymère préparé à partir de l'acide 2-acrylamido-2-méthylpropane-sulfonique (AMPS) ou l'un de ses sels de sodium ou d'ammonium, avec un ester de l'acide (méth)acrylique et :

- d'un alcool en $C_{10}$-$C_{18}$ oxyéthyléné par 8 moles d'oxyde d'éthylène (Genapol C-080® de la société Clariant),
- d'un alcool oxo en $C_{11}$ oxyéthyléné par 8 moles d'oxyde d'éthylène (Genapol UD-080® de la société Clariant),
- d'un alcool oxo en $C_{11}$ oxyéthyléné par 7 moles d'oxyde d'éthylène (Genapol UD-070® de la société Clariant),
- d'un alcool en $C_{12}$-$C_{14}$ oxyéthyléné par 7 moles d'oxyde d'éthylène (Genapol LA-070® de la société Clariant),
- d'un alcool en $C_{12}$-$C_{14}$ oxyéthyléné par 9 moles d'oxyde d'éthylène (Genapol LA-090® de la société Clariant),
- d'un alcool en $C_{12}$-$C_{14}$ oxyéthyléné par 11 moles d'oxyde d'éthylène (Genapol LA-110® de la société Clariant),
- d'un alcool en $C_{16}$-$C_{18}$ oxyéthyléné par 8 moles d'oxyde d'éthylène (Genapol T-080® de la société Clariant),
- d'un alcool en $C_{16}$-$C_{18}$ oxyéthyléné par 11 moles d'oxyde d'éthylène (Genapol T-110® de la société Clariant),
- d'un alcool en $C_{16}$-$C_{18}$ oxyéthyléné par 15 moles d'oxyde d'éthylène (Genapol T-150® de la société Clariant),
- d'un alcool en $C_{16}$-$C_{18}$ oxyéthyléné par 20 moles d'oxyde d'éthylène (Genapol T-200® de la société Clariant),
- d'un alcool en $C_{16}$-$C_{18}$ oxyéthyléné par 25 moles d'oxyde d'éthylène (Genapol T-250® de la société Clariant),
- d'un alcool en $C_{18}$-$C_{22}$ oxyéthyléné par 25 moles d'oxyde d'éthylène,
- d'un alcool en iso-$C_{16}$-$C_{18}$ oxyéthyléné par 25 moles d'oxyde d'éthylène.

Selon un mode préféré de réalisation, le polymère amphiphile est un copolymère d'AMPS et de méthacrylate d'alcool en $C_{16}$-$C_{18}$ comportant de 6 à 25 groupes oxyéthylénés, obtenus à partir d'acide méthacrylique ou d'un sel d'acide méthacrylique et d'un alcool en $C_{16}$-$C_{18}$ oxyéthyléné par 6 à 25 moles d'oxyde d'éthylène.

Le polymère amphiphile peut également être un copolymère d'AMPS et de méthacrylate d'alcool en $C_{12}$-$C_{14}$ comportant de 6 à 25 groupes oxyéthylénés, obtenus à partir d'acide méthacrylique ou d'un sel d'acide méthacrylique et d'un alcool en $C_{12}$-$C_{14}$ oxyéthyléné par 6 à 25 moles d'oxyde d'éthylène.

Comme polymères amphiphiles préférés selon la présente invention, on peut citer :

- le copolymère non réticulé obtenu à partir de 92,65 % en moles d'AMPS et de 7,35 % en moles de méthacrylate d'alcool en $C_{16}$-$C_{18}$ comportant 8 groupes oxyéthylénés (Genapol T-080®),
- le copolymère non réticulé obtenu à partir de 91,5 % en moles d'AMPS et de 8,5 % en moles de méthacrylate d'alcool en $C_{12}$-$C_{14}$ comportant 7 groupes oxyéthylénés (Genapol LA-070®).
- le copolymère réticulé obtenu à partir de 96,45 % en moles d'AMPS et de 3,55 % en moles de méthacrylate d'alcool

en $C_{16}$-$C_{18}$ comportant 25 groupes oxyéthylénés (Genapol T-250®), l'agent de réticulation est le triméthylol propane triacrylate,

- le copolymère réticulé obtenu à partir d'AMPS et de méthacrylate d'alcool en $C_{22}$ comportant 25 groupes oxyéthylénés ; ce polymère est vendu sous la dénomination Aristoflex HMB® par la société Clariant.

Ces copolymères sont appropriés pour donner des émulsions stables et se présentant sous des textures très variées, allant du fluide sprayable à la crème avec de très bonnes qualités cosmétiques.

De façon encore plus préférée, les polymères amphiphiles de l'invention sont choisis parmi :

- le copolymère non réticulé obtenu à partir de 92,65 % en moles d'AMPS et de 7,35 % en moles de méthacrylate d'alcool en $C_{16}$-$C_{18}$ comportant 8 groupes oxyéthylénés (Genapol T-080®),
- le copolymère non réticulé obtenu à partir de 91,5 % en moles d'AMPS et de 8,5 % en moles de méthacrylate d'alcool en $C_{12}$-$C_{14}$ comportant 7 groupes oxyéthylénés (Genapol LA-070®),
- et leurs mélanges.

b) <u>Les polymères dérivés d'acide acrylique</u>

Le polymère amphiphile selon l'invention peut être choisi parmi les dérivés d'acide acrylique. Ces polymères comprennent :

- de 80 à 99 % en moles de motif acide acide acrylique (AA) de formule (5) suivante :

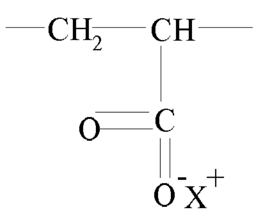

(5)

dans laquelle $X^+$ est un proton, un cation de métal alcalin, un cation alcalino-terreux ou l'ion ammonium ; et

- de 1 à 20 % en moles, et de préférence de 1 à 15 % en moles de motif de formule (6) suivante :

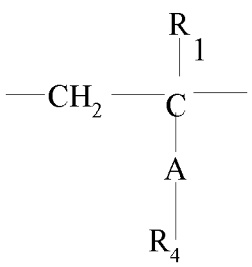

(6)

dans laquelle, $R_1$ désigne un atome d'hydrogène, un radical alkyle linéaire ou ramifié en $C_1$-$C_6$ (de préférence méthyle), A désigne un groupe ester ou amide ou un atome d'oxygène et $R_4$ désigne un alkyle linéaire ou ramifié comportant m atomes de carbone avec m allant de 6 à 30, de préférence de 10 à 25.

Comme polymères amphiphiles dérivés d'acide acrylique préférés selon la présente invention, on peut citer :

- le copolymère non réticulé obtenu à partir d'acide (méth)acrylique et de méthacrylate de stéareth-20, vendu sous la dénomination d'Aculyn 22® par la société Rohm and Haas,
- le copolymère non réticulé obtenu à partir d'acide (méth)acrylique et de méthacrylate de laureth-25, vendu sous la dénomination d'Aculyn 25® par la société Rohm and Haas,
- le copolymère non réticulé obtenu à partir d'acide (méth)acrylique et de méthacrylate de béheneth-25, vendu sous la dénomination d'Aculyn 28® par la société Rohm and Haas,
- le copolymère réticulé obtenu à partir d'acide (méth)acrylique et de vinyl néodécanoate, vendu sous la dénomination

d'Aculyn 38® par la société Rohm and Haas,

- le copolymère réticulé obtenu à partir d'acide (méth)acrylique et de méthacrylate de stéareth-20, vendu sous la dénomination d'Aculyn 88® par la société Rohm and Haas,
- les copolymères réticulés d'alkyl-$C_{10}$-$C_{30}$ acrylate et d'acide (meth)acrylique, comme les Pemulen TR1® et TR2® vendus par la société Novéon,
- le copolymère réticulé d'acide acrylique et d'iso-décanoate de vinyle, vendu sous la dénomination Stabylen 30®par la société 3V,
- les copolymères réticulés obtenus à partir d'acide (méth)acrylique et de $C_{10}$-$C_{30}$ alkyl acrylate, vendus sous la dénomination de Carbopol ETD 2020® et de Carbopol 1382®par la société Novéon,
- le copolymère non réticulé obtenu à partir d'acide (méth)acrylique et d'itaconate de stéareth-20, vendu sous la dénomination de Structure 2001® par la société National Starch,
- le copolymère non réticulé obtenu à partir d'acide (méth)acrylique et d'itaconate de céteth-20, vendu sous la dénomination de Structure 3001® par la société National Starch,
- le copolymère non réticulé obtenu à partir d'acide (méth)acrylique, d'aminoacrylate et de $C_{10}$-$C_{30}$ alkyl PEG 20 itaconate, vendu sous la dénomination de Structure Plus® par la société National Starch,
- le copolymère non réticulé obtenu à partir d'acide (méth)acrylique, de méthylacrylate et de diméthyl méta-isopropenyl benzyl isocyanate d'alcool éthoxylé, vendu sous la dénomination de Viscophobe DB 1000® par la société Amerchol.

Les polymères dérivés d'acide acrylique est en particulier un copolymère non réticulé obtenu à partir d'acide (méth)acrylique, de méthylacrylate et de diméthyl méta-isopropenyl benzyl isocyanate d'alcool éthoxylé.

c) <u>Les dérivés de polyéther</u>

Les polymères amphiphiles dérivés de polyéther préférés de l'invention sont des polyuréthanes hydrosolubles et notamment des composés polyéthylèneglycol (par exemple ayant de 45 à 160 motifs d'oxyde d'éthylène) portant aux extrémités une chaîne alkyle en $C_8$-$C_{20}$ via une liaison uréthane.
Parmi ceux-ci, on peut citer :

- le copolymère PEG-150 portant des extrémités stéaryle via des liaisons uréthanes, vendu sous la dénomination Aculyn 46® par la société Rohm and Haas,
- le copolymère PEG-150 portant des extrémités décyle via des liaisons uréthanes, vendu sous la dénomination Aculyn 44® par la société Rohm and Haas,
- le copolymère PEG-136 portant des extrémités stéaryle via des liaisons uréthanes, vendu sous la dénomination Nuvis FX 1100® par la société Elementis,
- le copolymère PEG-50 portant des extrémités stéaryle via des liaisons uréthanes, vendu sous la dénomination de Borchigel LW 44® par la société Borchers France.

Les polymères dérivés de polyéther peuvent également porter des chaînes grasses sans liaisons uréthane, comme par exemple le produit Pure Thix HH® vendu par la société Sud Chemie.
Les polymères dérivés de polyéther peuvent également être choisis parmi les copolymères à blocs d'oxyde d'éthylène et d'oxyde de propylène, de formule suivante :

$$HO(C_2H_4O)_x(C_3H_6O)_y(C_2H_4O)_zH$$

Dans laquelle x, y et z sont des nombres entiers tels que x+z va de 2 à 100 et y va de 14 à 60, et plus particulièrement parmi les copolymères à blocs de formule précédente ayant un HLB allant de 7 à 16.
Ces copolymères à blocs peuvent notamment être choisis parmi les polycondensats tribloc polyéthylène glycol / polypropylène glycol / polyéthylène glycol et notamment parmi ceux vendus sous les dénominations:

- le Pluronic L42® de formule précédente avec x = z = 3 et y = 18 (HLB = 8)
- le Pluronic P84® (nom INCI : POLOXAMER 184)de formule précédente avec x = z = 12 et y = 27 (HLB = 14)
- le Pluronic P103® (nom INCI : POLOXAMER 333) de formule précédente avec x = z = 12 et y = 40 (HLB = 9)
- le Pluronic P123® (nom INCI : POLOXAMER 184) de formule précédente avec x = z = 14 et y=48 (HLB = 8)
- le Pluronic L44® (nom INCI : POLOXAMER 124) de formule précédente avec x = z = 5 et y = 21 (HLB= 16).

d) <u>Les polymères amphiphiles issus de polymères naturels</u>

Les polymères amphiphiles hydrosolubles ou hydrodispersibles d'origine naturelle préférés de l'invention sont

par exemple choisis parmi les dérivés cellulosique, les dérivés du guar, les dérivés d'amidon et les dérivés de la gomme d'acacia modifiés par des chaînes grasses comportant de 6 à 30 atomes de carbone.

Parmi les dérivés cellulosiques modifiés par des chaînes grasses comportant de 6 à 30 atomes de carbone citons :

- les cétylhydroxyéthylcelluloses tels que le Natrosol CS Plus 330®' 430® et le Polysurf 67 CS® vendues par la société Hercules,
- les hydroxypropylméthylcelluloses modifiées par des chaînes stéaryloxyhydroxypropyl à un taux molaire compris entre 0,3 et 0,6 %, vendues sous les dénominations de Sangelose 60L® (masse molaire de l'ordre de 500 000 g/mole) et Sangelose 90L® (masse molaire de l'ordre de 900 000 g/mole) par la société Daido,
- l'hydroxyéthylcellulose quaternisée par l'époxyde de lauryl di-méthyl ammonium substitué, vendue sous la dénomination de Quadrisoft LM 200® par la société Amerchol,
- L'hydroxyéthylcellulose quaternisée et modifiée par des chaînes lauryle ou stéaryle, vendue sous la dénomination de Crodacel QM® (C12), QL® (C12) et QS® (C18) par la société Croda.

Parmi les dérivés du guar modifiés par des chaînes grasses comportant de 6 à 30 atomes de carbone citons l'hydroxypropylguar modifié par des chaînes béhénique, vendu sous la dénomination de Esaflor HM 22® par la société Lamberti.

Parmi les dérivés d'amidons modifiés par des chaînes grasses comportant de 6 à 30 atomes de carbone citons l'amidon de maïs estérifié par l'anhydride octenylsuccinique sous forme sel de sodium, vendu sous la dénomination de N-creamer 46® par la société National Starch,

Parmi les dérivés de la gomme d'acacia modifiés par des chaînes grasses comportant de 6 à 30 atomes de carbone, citons la gomme d'acacia modifiée par estérification contrôlée, vendue sous la dénomination de Ticamulsion A-2010® par la société Tic Gums.

Le polymère amphiphile issu de polymère naturel est en particulier une cétylhydroxyéthylcellulose.

Parmi les polymères hydrosolubles ou hydrodispersibles amphiphiles, on utilise de préférence :

- le copolymère non réticulé obtenu à partir de 92,65 % en moles d'AMPS et de 7,35 % en moles de méthacrylate d'alcool en $C_{16}$-$C_{18}$ comportant 8 groupes oxyéthylénés (Genapol T-080®),
- le copolymère non réticulé obtenu à partir de 91,5 % en moles d'AMPS et de 8,5 % en moles de méthacrylate d'alcool en $C_{12}$-$C_{14}$ comportant 7 groupes oxyéthylénés (Genapol LA-070®)
- le copolymère réticulé obtenu à partir d'acide (méth)acrylique et de méthacrylate de stéareth-20, vendu sous la dénomination d'Aculyn 88® par la société Rohm and Haas,
- les copolymères réticulés d'alkyl-$C_{10}$-$C_{30}$ acrylate et d'acide (meth)acrylique, comme les Pemulen TR1® et TR2® vendus par la société Novéon,
- le copolymère non réticulé obtenu à partir d'acide (méth)acrylique, de méthylacrylate et de diméthyl méta-isopropenyl benzyl isocyanate d'alcool éthoxylé, vendu sous la dénomination de Viscophobe DB 1000® par la société Amerchol
- le copolymère PEG-136 portant des extrémités stéaryle via des liaisons uréthanes, vendu sous la dénomination Nuvis FX 1100® par la société Elementis,
- le polymère cétylhydroxyéthylcellulose,
- et leurs mélanges

Les polymères amphiphiles plus particulièrement préférés sont

- le copolymère non réticulé obtenu à partir de 92,65 % en moles d'AMPS et de 7,35 % en moles de méthacrylate d'alcool en $C_{16}$-$C_{18}$ comportant 8 groupes oxyéthylénés (Genapol T-080®)
- le copolymère non réticulé obtenu à partir d'acide (méth)acrylique, de méthylacrylate et de diméthyl méta-isopropenyl benzyl isocyanate d'alcool éthoxylé, vendu sous la dénomination de Viscophobe DB 1000®par la société Amerchol
- le polymère cétylhydroxyéthylcellulose issu de polymère naturel
- et leurs mélanges.

*2) Polymère liposoluble ou lipodispersible amphiphile*

Par « polymères liposolubles ou lipodispersibles », on entend des polymères qui, introduits dans une huile ou un mélange huileux, à une concentration égale à 1 %, sous agitation à 50 °C pendant 48 heures, conduisent après retour à la température ambiante à une solution macroscopiquement homogène dont la transmittance de la lumière, à une longueur d'onde égale à 500 nm, à travers un échantillon de 1 cm d'épaisseur, est d'au moins 10 %, de préférence d'au moins 20 %.

Les polymères liposolubles ou lipodispersibles amphiphiles de l'invention peuvent permettre de stabiliser des émulsions à phase continue huileuse

Ils peuvent être constitués d'au moins une partie polaire et d'au moins une partie apolaire. Ils peuvent présenter une structure de type bloc ou peigne.

Les polymères liposolubles ou lipodispersibles amphiphiles peuvent être choisis parmi les dérivés de polyoléfines, les diméthicones copolyols, les élastomères réticulés d'organopolysiloxanes émulsionnants, les dérivés de polyéthers, les dérivés de polyhydroxystéarate et leurs mélanges.

a. Les dérivés de polyoléfines

Les polymères amphiphiles dérivés de polyoléfines peuvent permettre de fabriquer des émulsions eau-dans-huile stables au stockage et adaptée aux applications cosmétiques, dermatologiques et/ou pharmaceutiques. En particulier, ils permettent de fabriquer des émulsions eau-dans-huile très riches en phase aqueuse (plus de 80 % en poids) et stables, ou bien des émulsions eau-dans-huile de faible viscosité et stables. Ces tensio-actifs présentent ainsi des avantages par rapport aux agents émulsionnants couramment utilisés pour stabiliser les émulsions eau dans huile, en particulier les alkyls polyglycérol, les alkyls POE, les alkyls de sorbitane, les sels métalliques d'acides gras et les tensioactifs siliconés. En effet pour ces émulsionnants, la teneur en phase aqueuse est en général inférieure à 80 % en poids, les concentrations en tensio-actifs sont élevées pour assurer une bonne stabilité des émulsions et la phase grasse comprend une proportion majoritaire d'huiles siliconées lorsque des tensio-actifs siliconés sont utilisés. D'autre part, les émulsions eau dans huile stabilisées par un tensio-actif dérivé de polyoléfines possèdent des propriétés cosmétiques particulièrement bonnes, avec un toucher léger et frais, sans être siliconé.

La partie apolaire peut être choisie parmi les polyoléfines telles que polymères et/ou copolymères d'éthylène, de propylène, de 1-butène, d'isobutène, de 1-pentène, de 2-méthyl-1-butène, de 3-methyl-1-butène, de 1-héxène, de 1-heptène, de 1-octène, de 1-décène, de 1-undécène, de 1-dodécéne, de 1-tridécène, de 1-tetradécène, de 1-pentadécène, de 1-hexadécéne, de 1-heptedécène et de 1-octadécéne. Les chaînes de polymères sont hydrogénés ou non. Elles sont constituées d'au moins 40 carbones, et de préférence de 60 à 700 carbones.

La partie polaire des polymères amphiphiles de l'invention peut être anionique, cationique, non ionique, zwitterionique ou amphotère. Elle est par exemple constituée de dérivés acryliques, de polyalkylènes glycols ou de polyalkylène imine. Les polymères amphiphiles à partie polaire acide carboxylique sont par exemple issus de la réaction d'une polyoléfine avec les acides carboxyliques tels que l'acide maléique, l'anhydride maléique, l'acide fumarique, l'acide itaconique, l'acide citraconique, l'acide mesaconique, l'acide aconitique.... De préférence, leur partie polaire est constituée par l'acide ou l'anhydride succinique, leurs dérivés esters ou amides, les sels d'ions alcalins, alcalino-terreux, ou organiques correspondants, ou bien encore par du polyoxyéthylène.

Les polymères liposolubles ou lipodispersibles amphiphiles de l'invention sont par exemple des polymères diblocs polyisoprène-polyoxyéthylène ou poly(éthylène-co-propylène)-polyoxyéthylène décrits dans la publication de Allgaier, Poppe, Willner, Richter (Macromolecules, 1997, 30, p.1582-1586).

Les polymères amphiphiles de l'invention peuvent également être choisis parmi les dérivés polyoléfines d'acide succinique décrits dans les brevets US 4 234 435, US 4 708 753, US 5 129 972, US 4 931 110, GB 2 156 799 et US 4 919 179. La partie polyoléfine peut être constituée de polyisobutylène, hydrogéné ou non. L'anhydride ou l'acide succinique peuvent être modifiés par des alcools, des amines, des alcanols amines ou des polyols, ou encore se trouver sous forme de sels d'ions alcalins ou alcalino-terreux, ou encore d'ions organiques comme les ions diéthanolammonium ou triéthanolammonium. On peut citer notamment les polyisobutylène à terminaison succinique modifiée, tels que les produits commercialisés sous les dénominations L2724®, L2721®, L2722®, OS156565® et Lubrizol 5603® par la société Lubrizol.

Un autre exemple de polymère amphiphile utilisable dans l'invention est le produit de la réaction de l'anhydride maléique avec du polyisobutylène, tel que le Glissopal SA® commercialisé par BASF.

b. Les diméthicones copolyols

Parmi les polymères amphiphiles adaptés pour stabiliser les émulsions à phase continue huileuse, citons les dimethicone copolyols tels que le mélange de cyclomethicone et de dimethicone copolyol, vendu sous la dénomination DC 5225 C® par la société Dow Corning, et les alkyl-dimethicone copolyols tels que le Laurylmethicone copolyol vendu sous la dénomination Dow Corning 5200 Formulation Aid® par la société Dow Corning et le Cetyl dimethicone copolyol vendu sous la dénomination ABIL EM 90® par la société Goldschmidt, ou le mélange polyglycéryl-4 isostéarate/cétyl diméthicone copolyol/hexyllaurate vendu sous la dénomination ABIL WE 09® par la société Goldschmidt, ou le polydiméthylméthylsiloxane oxyéthyléné vendu sous la dénomination DC SH 3773 M® par la société Dow Corning.

c. Les élastomères réticulés d'organopolysiloxanes émulsionnants

On peut aussi utiliser un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004 et tel que celui commercialisé sous la référence KSG 21® par la société Shin Etsu.
On peut également utiliser les élastomères de silicones émulsionnants tels que ceux vendus sous les dénominations KSG-210, KSG-310, KSG-320, KSG-330, KSG-440, KSG-710, KSG-830, KSG-840 par la société SHIN-ETSU.

d. Les dérivés de polyéther

Le polymère dérivé de polyéther peut également être choisi parmi les copolymères à blocs d'oxyde d'éthylène et d'oxyde de propylène, de formule suivante :

$$HO(C_2H_4O)_x(C_3H_6O)_y(C_2H_4O)_zH$$

Dans laquelle x, y et z sont des nombres entiers tels que x+z va de 2 à 100 et y va de 14 à 60, et plus particulièrement parmi les copolymères à blocs de formule précédente ayant un HLB allant de 2 à 6.
Ces copolymères blocs peuvent être notamment choisis parmi les polycondensats tribloc polyéthylène glycol / polypropylène glycol / polyéthylène glycol. vendus sous les dénominations "SYNPERONIC" comme les « SYNPERONIC® PE/L81 » (nom INCI : POLOXAMER 231) ; «SYNPERONIC® PE/L92» (nom INCI : POLOXAMER 282) par la société UNIQEMA.

e. Les dérivés de Polyhydroxystéarate

Le polymère amphihile selon l'invention peut être choisi parmi les polymères dérivés de polyhydroxystéarate possèdent une structure de type multiblocs. De préférence, ces polymères sont constitués de trois blocs B-A-B où B est un polyhydroxystéarate et A est un polyéther. Citons le copolymère de polyéthylène glycol (30 OE) et d'acide 12-hydroxy stéarique (nom INCI : PEG-30 dipolyhydroxystéarate) vendu sous la dénomination d'Arlacel P135® par la société Uniqema.
Le polymère amphiphile présent dans le kit ou compositions selon l'invention est de préférence choisi parmi les polymères amphiphiles hydrosolubles ou hydrodispersibles, notamment parmi ceux décrits précédemment.
Le polymère amphiphile peut être présent dans l'une au moins des première et seconde compositions selon l'invention, en particulier les émulsions à phase continue aqueuse, en une teneur comprise entre 0,01 et 10 %, de préférence entre 0,05 et 5 %, voire entre 0,25 et 3 %, en poids par rapport au poids total de la composition.
Le polymère amphiphile peut être présent dans les compositions selon l'invention, en particulier les émulsions à phase continue huileuse, en une teneur comprise entre 0,1 et 10 %, voire entre 1 et 5 %, en poids par rapport au poids total de la composition.
La concentration en polymère amphiphile et en composé X et/ou en composé Y dans les compositions selon l'invention peut être telle que le rapport massique entre le polymère amphiphile et le composé X et/ou composé Y est compris entre 0,01 et 1, en particulier entre 0,05 et 0,2
Optionnellement, sans caractère obligatoire, il est possible d'ajouter, afin de faciliter l'émulsification de la phase huileuse, au moins un co-tensioactif différent du polymère amphiphile décrit précédemment.

**Tensioactifs**

Le (ou les) co-tensioactif est par exemple utilisé à un taux inférieur à 50 % par rapport au poids du polymère amphiphile selon l'invention.
Ce tensioactif peut être non ionique, amphotère ou cationique et de préférence anionique. On peut citer par exemple comme tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en $C_8$-$C_{24}$, et leurs dérivés oxyalkylénés ; les esters de polyéthylène glycol et d'acide gras en $C_8$-$C_{24}$, et leurs dérivés oxyalkylénés ; les esters de sorbitol et d'acide gras en $C_8$-$C_{24}$, et leurs dérivés oxyalkylénés ; les esters de sucre (sucrose, glucose, alkylglucose) et d'acide gras en $C_8$-$C_{24}$, et leurs dérivés oxyalkylénés ; les éthers d'alcools gras ; les éthers de sucre et d'alcools gras en $C_8$-$C_{24}$, et leurs mélanges.
Comme ester de glycéryle et d'acide gras, on peut citer notamment le stéarate de glycéryle (mono-, di- et/ou tri-stéarate de glycéryle) (nom CTFA : glyceryl stearate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stearate), le monostéarate de polyéthylène glycol 100 OE (nom CTFA : PEG-100 stearate) et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom CTFA : glyceryl stearate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate) ; l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesquiisostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à 22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic et leurs mélanges.

Parmi les esters ou éthers de polyols, on peut citer plus particulièrement les esters de glycérol, les esters de polyéthylène glycol, les esters de sorbitan, et leurs mélanges.

Convient aussi à l'invention le mono-isostéarate de glycéryle, tel que le produit commercialisé sous la dénomination de Peceol Isostarique® par la société Gattefosse, l'isostéarate de PEG-8 tel que le produit commercialisé sous la dénomination Prisorine 3644® par la société Uniqema, l'isostéarate polyglycérolé (4 moles) vendu sous la dénomination Isolan GI34® par la société Goldschmidt, le diisostéarate polyglycérolé (3 moles) vendu sous la dénomination Lameform TGI® par la société Cognis et le distéarate polyglycérolé (2 moles) vendu sous la dénomination Emalex PGSA® par la société Nihon emulsion.

L'isostéarate de sorbitan tel que le produit commercialisé sous la dénomination Arlacel 987® par la société Uniqema, l'isostérate de sorbitan et de glycérol, tel que le produit commercialisé sous la dénomination Arlacel 986® par la société Uniqema, le sesquioleate de sorbitane tel que le produit vendu sous la dénomination Arlacel 83V® par la

société Uniqema, le laurate de sorbitane, l'oléate de sorbitane et le trioléate de sorbitane tels que les produits commercialisés sous la dénomination Span 20®, Span 80V® et Span 85V® par la société Uniqema sont également susceptibles d'être mis en oeuvre.

Comme esters de sucre, on peut citer par exemple l'isostéarate de méthylglucose.

Selon un mode préféré de l'invention, le coémulsionnant est choisi parmi les esters de glycéryle.

Les compositions de l'invention peuvent contenir tous les additifs habituellement utilisés en cosmétique et trouveront des applications dans le domaine du soin, du maquillage, des produits solaires et de l'hygiène corporelle.

Ainsi, la phase aqueuse des compositions peut contenir des glycols tels que le dipropylène glycol, le glycérol et le butylène glycol, des filtres UV hydrosolubles ou hydrodispersibles organiques et minéraux, des actifs, des sels, des charges, Comme actifs, on peut utiliser notamment les vitamines (A, C, E, K, PP...) seules ou en mélange ainsi que leurs dérivés, les agents kératolytiques et/ou desquamants (acide salicylique et ses dérivés, alpha-hydroxyacides, acide ascorbique et ses dérivés), les agents anti-inflammatoires, les agents apaisants, les agents dépigmentants, les agents tenseurs tels que les polymères synthétiques, les protéines végétales, les polysaccharides d'origine végétale sous forme ou non de microgels, les amidons, les dispersions de cires, les silicates mixtes et les particules colloïdales de charges inorganiques ; les agents matifiants, ou encore les agents anti-rides et leurs mélanges. La phase huileuse peut contenir des gélifiants lipophiles, des cires, des particules organiques ou minérales ou encore des filtres solaires lipophiles ou lipodispersibles organiques et minéraux.

Selon la fluidité de la composition que l'on souhaite obtenir, on peut incorporer dans la composition, un ou plusieurs gélifiants, notamment hydrophiles, c'est-à-dire solubles ou dispersibles dans l'eau.

Comme gélifiants hydrophiles, on peut citer en particulier les polymères épaississants hydrosolubles ou hydrodispersibles. Ceux-ci peuvent notamment être choisis parmi : les polymères carboxyvinyliques modifiés ou non, tels que les produits commercialisés sous les dénominations Carbopol® (nom CTFA : carbomer) par la société Noveon ; les polyacrylates et polyméthacrylates tels que les produits vendus sous les dénominations de Lubrajel® et Norgel® par la société GUARDIAN ou sous la dénomination Hispagel® par la société HISPANO CHIMICA ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination Hostacerin AMPS® (nom CTFA : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305® (nom C.T.F.A. : Polyacrylamide / $C_{13-14}$ Isoparaffin / Laureth-7) et sous le nom de SIMULGEL 600® (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose muicrocristalline, la carboxyméthylcellulose, l'hydroxyméthylcellullose et l'hydroxypropylcellulose ; et leurs mélanges.

Comme gélifiants lipophiles, on peut citer par exemple les argiles modifiées telles que le silicate de magnésium modifié (bentone gel VS38® de RHEOX), l'hectorite modifiée par le chlorure de distéaryl diméthyl ammonium (nom CTFA : Disteardimonium hectorite) commercialisé sous la dénomination bentone 38 CE® par la société RHEOX.

Les compositions selon l'invention peuvent en outre comprendre des charges.

Comme charges, on peut citer par exemple, les particules de polyamide (Nylon®) et notamment celles vendues sous les dénominations ORGASOL® par la société Arkema ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP® ; les microsphères de polyméthacrylate de méthyle, commercialisées sous la dénomination MICROSPHERE M-100® par la société Matsumoto ou sous la dénomination COVABEAD LH85® par la société Wackherr ; les poudres de copolymère éthylène-acrylate, comme celles commercialisées sous la dénomination FLOBEADS® par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL® par la société Kemanord Plast sous les références 551 DE 12® (granulométrie d'environ 12 $\mu$m et masse volumique 40 kg/$m^3$), 551 DE 20® (granulométrie d'environ 30 $\mu$m et masse volumique 65 kg/$m^3$), 551 DE 50® (granulométrie d'environ 40 $\mu$m), ou les microsphères commercialisées sous la dénomination MICROPEARL F 80 ED® par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO® par la société National Starch ; les microbilles de résine de silicone telles que celles commercialisées sous la dénomination TOSPEARL® par la société Toshiba Silicone, notamment TOSPEARL 240® ; et leurs mélanges.

L'une au moins des compositions d'un kit selon l'invention peut également comprendre au moins une matière colorante.

Pour une application en particulier au soin ou au maquillage des peaux grasses, les compositions selon l'invention peuvent comprendre au moins un actif choisi parmi : les agents desquamants, les agents anti-séborrhéiques,

les agents anti-microbiens, et les agents apaisants.

Pour une application en particulier au soin ou au maquillage des peaux âgées, les compositions selon l'invention peuvent comprendre au moins un actif choisi parmi : les agents desquamants ou hydratants ; les agents dépigmentants ou anti-pigmentants ; les agents anti-glycation ; les agents anti-NO ; les agents stimulant la synthèse de macromolécules dermiques ou épidermiques et/ou empêchant leur dégradation ; les agents stimulant la prolifération des fibroblastes ou des kératinocytes et/ou la différenciation des kératinocytes ; les agents myorelaxants ou dermo-décontractants ; les agents anti-radicalaires ou anti-pollution ; les agents tenseurs ; et les agents agissant sur la micro-circulation.

Les compositions de ce type peuvent avoir la forme d'un produit de soin ou de maquillage, d'un produit capillaire, d'un produit solaire ou d'un produit d'hygiène corporelle et être conditionnée par exemple sous forme de crème en pot ou de fluide en tube ou en flacon pompe.

Les compositions selon l'invention ou utilisées pour le procédé selon l'invention peuvent se présenter sous la forme d'une composition de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, composition de bronzage artificiel); une composition après-rasage.

Le kit selon l'invention peut notamment être utile à titre de composition de revêtement de la peau du corps ou du visage, et plus particulièrement de composition de maquillage et /ou de soin de la peau du corps ou du visage.

Les compositions selon l'invention peuvent être utilisées pour le maquillage de la peau, du corps et du visage, des lèvres, des cils et/ou des ongles selon la nature des ingrédients utilisés.

En particulier, la ou les compositions selon l'invention peuvent se présenter sous forme d'un fond de teint, d'un bâton ou d'une pâte de rouge à lèvres, de produit anti-cernes ou contour des yeux, d'eye-liner, d'ombre à paupières, de produit du maquillage du corps ou encore un produit de coloration de la peau.

L'homme du métier pourra choisir la forme galénique appropriée, ainsi que sa méthode de préparation, sur la base de ses connaissances générales, en tenant, d'une part, de la nature des constituants utilisés, notamment de leur solubilité dans les support, et, d'autre part, de l'application envisagée pour chaque composition.

L'invention est illustrée plus en détails, de manière non limitative, par les exemples décrits ci-après. Sauf indications contraires, les quantités indiquées sont exprimées en pourcentage massique.

## Exemples

**[0258]** - Dans les exemples de compositions décrites ci-après, on utilise, à titre de composés X et Y, la combinaison des mélanges A et B suivants préparés par la société Dow Corning :

**MELANGE A :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| Ingrédient (Nom INCI) | N°CAS | Teneurs (%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

- Les copolymères amphiphiles des exemples suivants sont fournis soit sous forme de poudre soit en solution aqueuse. Lorsqu'ils sont sous forme de poudre, on les solubilise dans l'eau pendant 30 minutes sous agitation à 25 °C ; la solution obtenue est macroscopiquement homogène.
Lorsqu'ils sont en solution aqueuse, on les dilue avec de l'eau pendant 30 minutes sous agitation à 25 °C ; la solution obtenue est macroscopiquement homogène.
- Les émulsions des exemples suivants sont préparées par introduction lente de la phase huileuse dans la phase

aqueuse sous agitation à l'aide d'un homogénéisateur de type Moritz sous une vitesse d'agitation de 4000 RPM pendant 15 minutes.
- La brillance des émulsions après séchage à l'état de film est mesurée selon le protocole *in vitro* suivant.

On applique un film de la composition à tester sur une carte de contraste (Prufkarte type 24/5 - 250 $cm^2$ commercialisée par la société Erichsen) à l'aide d'un tire-film (épaisseur humide 50 microns). Dans le cas d'un produit conforme à l'invention, on mélange de façon extemporanée les premières et secondes compositions contenant respectivement les composés X et Y en une proportion pondérale 50/50 puis on applique ce mélange sur la carte. On sèche ensuite la composition pendant 24 heures à une température de 37 C°.

Les mesures de brillance sont réalisées à l'aide du brillancemètre micro TRI gloss BYK Gardner. La mesure de brillance est réalisée en mesurant la lumière réfléchie suivant un angle de 20° par rapport à la normale au plan de l'échantillon.

**Exemple 1 :**

Emulsions huile dans eau comprenant un polymère amphiphile à base d'AMPS

Les compositions des émulsions sont les suivantes :

Le polymère amphiphile utilisé est hydrosoluble et a une structure en peigne. Il est obtenu à partir de 92,65 % en moles d'AMPS et de 7,35 % en moles de méthacrylate d'alcool en $C_{16}$-$C_{18}$ comportant 8 groupes oxyéthylénés (Genapol T080) et est fourni par la société Clariant.

| *Phase* | *Nom* | ***Concentration (% massique)*** | | | |
|---|---|---|---|---|---|
| | | Emulsion 1a | Emulsion 1b | Emulsion 1c | Mélange 1a+1b |
| A | Eau distillée | 78,296 | 78,296 | 88,296 | 78,296 |
| | Conservateur | 1 | 1 | 1 | 1 |
| | Triéthanolamine | 0,004 | 0,004 | 0,004 | 0,004 |
| | Copolymère d'AMPS et de méthacrylate d'alcool en $C_{16}$-$C_{18}$ comportant 8 groupes oxyéthylénés[1] | 0,5 | 0,5 | 0,5 | 0,5 |
| | Acide poly-acrylamidométhyl propane sulfonique neutralisé partiellement à l'ammoniaque et hautement réticulé[2] | 0,2 | 0,2 | 0,2 | 0,2 |
| B | Huile de Parleam | 5 | 5 | 5 | 5 |
| | Cyclohexadiméthylsiloxane | 5 | 5 | 5 | 5 |
| | Mélange A | 10 | 0 | 0 | 5 |
| | Mélange B | 0 | 10 | 0 | 5 |
| Total | | 100 | 100 | 100 | 100 |
| [1] : Genapol T080® de Clariant<br>[2] : Hostacerin AMPS de Clariant | | | | | |

Les émulsions 1a, 1b, 1c et le mélange 1a +1b sont de belles crèmes blanches.
Seules les émulsions 1a, 1b et 1c manifestent un effet brillant significatif.
Ceci est confirmé par les résultats du test de brillance soumis ci-après.

Mesure de la brillance des émulsions de l'exemple 1.

| | Emulsion 1a | Emulsion 1b | Emulsion 1c | Mélange 1a + 1b |
|---|---|---|---|---|
| **Brillance** | 181,7 +/- 0,9 | 177,9 +/- 2,8 | 183,6 +/- 1,5 | 11,3 +/- 2,8 |

**Exemple 2 :**

Emulsions huile dans eau comprenant un polymère amphiphile à base d' hydroxyéthylcellulo se

Le copolymère utilisé est hydrosoluble et a une structure en peigne. Il s'agit d'une cétylhydroxyéthylcellulose vendue sous la dénomination de Natrosol CS Plus 330 par la société Hercules.

Les compositions des émulsions sont les suivantes :

| *Phase* | *Nom* | ***Concentration (% massique)*** | | | |
|---|---|---|---|---|---|
| | | Emulsion 2a | Emulsion 2b | Emulsion 2c | Mélange 2a + 2b |
| A | Eau distillée | 77,8 | 77,8 | 87,8 | 77,8 |
| | Conservateur | 1 | 1 | 1 | 1 |
| | Cetylhydroxyethylcellulose[5] | 1 | 1 | 1 | 1 |
| | Acide poly-acrylamidométhyl propane sulfonique neutralisé partiellement à l'ammoniaque et hautement réticulé[2] | 0,2 | 0,2 | 0,2 | 0,2 |
| B | Huile de Parleam | 5 | 5 | 5 | 5 |
| | Cyclohexadiméthylsiloxane | 5 | 5 | 5 | 5 |
| | Mélange A | 10 | 0 | 0 | 5 |
| | Mélange B | 0 | 10 | 0 | 5 |
| Total | | 100 | 100 | 100 | 100 |
| [5]: Natrosol CS Plus 330® de la société Hercules<br>[2] : Hostacerin AMPS® de Clariant | | | | | |

Mesure de la brillance des émulsions de l'exemple 2.

| | Emulsion 2a | Emulsion 2b | Emulsion 2c | Mélange 2a + 2b |
|---|---|---|---|---|
| **Brillance** | 94,1 +/- 14,9 | 80,1 +/- 6,3 | 169,3 +/- 4,9 | 9,3 +/- 2,4 |

Ces données montrent que le mélange d'émulsions selon l'invention qui correspond à l'application de l'émulsion 2a et de l'émulsion 2b de l'exemple 2 conduit à un film dont la brillance après séchage est réduite de façon très importante par rapport aux films obtenus à partir des émulsions 2a et 2b seules ou encore 2c.

**Exemple 3**

Emulsions huile dans eau comprenant un polymère amphiphile en peigne à base d'acide acrylique

Le copolymère amphiphile utilisé est le Viscophobe DB 100®. Il est hydrosoluble et a une structure en peigne. Il s'agit d'un copolymère non réticulé obtenu à partir d'acide (méth)acrylique, de méthylacrylate et d'isocyanate d'alcool éthoxylé de diméthyle méta-isopropenyle benzyle.

Les compositions des émulsions sont les suivantes :

| *Phase* | *Nom* | ***Concentration (% massique)*** | | | |
|---|---|---|---|---|---|
| | | Emulsion 3a | Emulsion 3b | Emulsion 3c | Mélange 3a +3b |
| A | Eau distillée | 71,8 | 71,8 | 81,8 | 71,8 |
| | Conservateur | 1 | 1 | 1 | 1 |
| | Triéthanolamine | 1,2 | 1,2 | 1,2 | 1,2 |
| | Polyacrylate-3 à 25% MA[7] | 6 | 6 | 6 | 6 |

(suite)

| Phase | Nom | Concentration (% massique) | | | |
|---|---|---|---|---|---|
| | | Emulsion 3a | Emulsion 3b | Emulsion 3c | Mélange 3a +3b |
| B | Huile de Parleam | 5 | 5 | 5 | 5 |
| | Cyclohexadiméthylsiloxane | 5 | 5 | 5 | 5 |
| | Mélange A | 10 | 0 | 0 | 5 |
| | Mélange B | 0 | 10 | 0 | 5 |
| Total | | 100 | 100 | 100 | 100 |
| 7 : Viscophobe DB 100® d'Amerchol | | | | | |

Mesure de la brillance des émulsions de l'exemple 3.

| | Emulsion 3a | Emulsion 3b | Emulsion 3c | Mélange 3a + 3b |
|---|---|---|---|---|
| **Brillance** | 163,7 +/- 0,3 | 154 +/- 4,3 | 144,4 +/- 2,9 | 21,1 +/- 7,2 |

Ces données montrent que le mélange d'émulsions selon l'invention qui correspond à l'application de l'émulsion 3a et de l'émulsion 3b de l'exemple 3 conduit à un film dont la brillance après séchage est réduite de façon très importante par rapport aux films obtenus à partir des émulsions 3a et 3b seules ou encore 3c.

**Revendications**

1. Kit cosmétique utile pour le soin et/ou le maquillage de matière(s) kératinique(s), notamment de la peau, comprenant au moins deux compositions différentes et conditionnées séparément avec l'une au moins des compositions étant de type émulsion et comprenant au moins un polymère amphiphile, le kit comprenant un ou plusieurs composés X, un ou plusieurs composés Y, et au moins un catalyseur, avec au moins un des composés X et Y étant siliconé et lesdits composés X et Y étant susceptibles de réagir ensembles par une réaction d'hydrosylilation lorsqu'ils sont mis en contact les uns avec les autres en présence d'un catalyseur, et dans lequel les composés X, Y et le catalyseur ne sont pas présents simultanément dans la même composition.

2. Kit selon la revendication 1 comprenant au moins :

   i. une première composition contenant, dans un milieu physiologiquement acceptable, au moins un composé X et
   ii. une seconde composition contenant, dans un milieu physiologiquement acceptable, au moins un composé Y,

   avec l'une au moins desdites première et seconde compositions étant sous la forme d'une émulsion et contenant au moins un polymère amphiphile, et l'une au moins desdites première et seconde compositions contenant en outre au moins un catalyseur.

3. Kit selon la revendication précédente, dans lequel ledit polymère amphiphile est présent dans l'une au moins des première ou seconde compositions en une teneur comprise entre 0,01 et 10 %, de préférence entre 0,05 et 5 %, voire entre 0,25 et 3%, en poids par rapport au poids de la composition.

4. Kit selon la revendication 1, 2 ou 3, dans lequel le polymère amphiphile est hydrosoluble ou hydrodispersible.

5. Kit selon la revendication 4, dans lequel ledit polymère hydrosoluble ou hydrodispersible est choisi parmi les polymères dérivés de l'acide acrylamido-2-méthylpropane sulfonique (AMPS), les polymères dérivés d'acide acrylique, les dérivés de polyéthers, les polymères amphiphiles issus de polymères naturels, et leurs mélanges.

6. Kit selon la revendication 5, dans lequel le polymère dérivé d'AMPS est choisi parmi les copolymères d'AMPS et de méthacrylate d'alcool en $C_{16}$-$C_{18}$ comportant de 6 à 25 groupes oxyéthylénés, obtenus à partir d'acide méthacrylique ou d'un sel d'acide méthacrylique et d'un alcool en $C_{16}$-$C_{18}$ oxyéthyléné par 6 à 25 moles d'oxyde d'éthylène.

**7.** Kit selon la revendication 5, dans lequel le polymère dérivé d'acide acrylique est un copolymère non réticulé obtenu à partir d'acide (méth)acrylique, de méthylacrylate et de diméthyl méta-isopropenyl benzyl isocyanate d'alcool éthoxylé.

**8.** Kit selon la revendication 5, dans lequel le dérivé de polyéther est choisi parmi les composés polyéthylèneglycol portant aux extrémités une chaîne alkyle en $C_8$-$C_{20}$ via une liaison uréthane et les copolymères à blocs d'oxyde d'éthylène et d'oxyde de propylène, de formule suivante :

$$HO(C_2H_4O)_x(C_3H_6O)_y(C_2H_4O)_zH$$

dans laquelle x, y et z sont des nombres entiers tels que x+z va de 2 à 100 et y va de 14 à 60.

**9.** Kit selon la revendication 5, dans lequel le polymère amphiphile issu de polymères naturels est choisi parmi les dérivés cellulosique, les dérivés du guar, les dérivés d'amidon et les dérivés de la gomme d'acacia modifiés par des chaînes grasses comportant de 6 à 30 atomes de carbone.

**10.** Kit selon l'une quelconque des revendications précédentes, dans lequel le polymère amphiphile est liposoluble ou lipodispersible.

**11.** Kit selon la revendication précédente, dans lequel le polymère liposoluble ou lipodispersible est choisi parmi les dérivés de polyoléfines, les diméthicones copolyols, les élastomères réticulés d'organopolysiloxanes émulsionnants, les dérivés de polyéthers, les dérivés de polyhydroxystéarate et leurs mélanges.

**12.** Kit selon l'une quelconque des revendications précédentes, **caractérisé en ce que** le composé X est choisi parmi les composés siliconés comprenant au moins deux groupements aliphatiques insaturés.

**13.** Kit selon la revendication 12 dans lequel le composé X est un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal).

**14.** Kit selon la revendication 13, **caractérisé en ce que** le composé X est porteur d'au moins un groupe polaire.

**15.** Kit selon l'une des revendications précédentes, **caractérisé en ce que** le composé X est choisi parmi les polyorganosiloxanes comprenant au moins deux groupements aliphatiques insaturés liés chacun à un atome de silicium.

**16.** Kit selon l'une des revendications précédentes, **caractérisé en ce que** le composé X est choisi parmi les polyorganosiloxanes comprenant des unités siloxanes de formule :

$$R_mR'SiO_{\frac{(3-m)}{2}} \quad (I)$$

dans laquelle :

- R représente un groupe hydrocarboné monovalent, linéaire ou cyclique, comprenant de 1 à 30 atomes de carbone,
- m est égal à 1 ou 2 et
- R' représente :
    - un groupement hydrocarboné aliphatique insaturé comprenant de 2 à 10, de préférence de 3 à 5 atomes de carbone ou
    - un groupement hydrocarboné cyclique insaturé comprenant de 5 à 8 atomes de carbone.

**17.** Kit selon la revendication précédente dans lequel le polyorganosiloxane de formule (I) est tel que R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical

alkyle comprenant de 1 à 4 atomes de carbone, de préférence un atome d'hydrogène.

**18.** Kit selon la revendication 16 ou 17, **caractérisé en ce que** R représente un radical alkyle comprenant de 1 à 10 atomes de carbone ou encore un groupement phényle, et de préférence un radical méthyle, et R' est un groupe vinyle.

**19.** Kit selon l'une des revendications 13 à 18, **caractérisé en ce que** les polyorganosiloxanes comprennent en outre des unités de formule :

$$R_nSiO_{\frac{(4-n)}{2}} \quad (II)$$

dans laquelle R représente un groupe hydrocarboné monovalent, linéaire ou cyclique, comprenant de 1 à 30 atomes de carbone, et n est égal à 1, 2 ou 3.

**20.** Kit selon l'une quelconque des revendications 1 à 11, **caractérisé en ce que** le composé X est choisi parmi les oligomères ou polymères organiques, les oligomères ou polymères hybrides organique/silicone, lesdits oligomères ou polymères portant au moins 2 groupements aliphatiques insaturés réactifs.

**21.** Kit selon l'une des revendications précédentes, dans lequel le composé Y comprend au moins deux groupes Si-H libres.

**22.** Kit selon l'une des revendications précédentes, **caractérisé en ce que** le composé Y est choisi parmi les polyorganosiloxanes comprenant au moins une unité alkylhydrogènosiloxane de formule suivante :

$$R_pHSiO_{\frac{(3-p)}{2}} \quad (III)$$

dans laquelle :

R représente un groupe hydrocarboné monovalent, linéaire ou cyclique, comprenant de 1 à 30 atomes de carbone ou un groupe phényle, et p est égal à 1 ou 2.

**23.** Kit selon la revendication précédente, dans lequel le composé Y est tel que les radicaux R représentent un groupement alkyle en $C_1$-$C_{10}$, de préférence méthyle.

**24.** Kit selon l'une des revendications 21 à 23, dans lequel Y est un polyorganosiloxane comprenant au moins deux unités alkylhydrogènosiloxane de formule $-(H_3C)(H)Si-O-$ et comprenant éventuellement des unités $-(H_3C)_2SiO-$.

**25.** Kit selon l'une des revendications précédentes, dans lequel le catalyseur est un catalyseur à base de platine ou d'étain.

**26.** Kit selon la revendication précédente, **caractérisé en ce que** le catalyseur est présent en une teneur allant de 0,0001 % à 20 % en poids par rapport au poids total de la composition le comprenant.

**27.** Kit selon l'une des quelconque des revendications 1 à 21, **caractérisé en ce que** le composé X est un polydiméthylsiloxane à groupements vinyliques terminaux et le composé Y est un polyméthylhydrogénosiloxane.

**28.** Kit selon l'une quelconque des revendications précédentes, dans lequel le composé X est porteur d'au moins un groupe polaire susceptible de former une liaison hydrogène avec les matières kératiniques.

**29.** Kit selon l'une quelconque des revendications précédentes comprenant dans au moins une des compositions une charge choisie parmi la silice ou la silice traitée en surface.

**30.** Kit selon l'une des revendications précédentes, **caractérisé en ce que** le composé X présente une masse moléculaire moyenne en poids (Mw) allant de 150 à 1 000 000, de préférence de 200 à 800 000, de préférence encore de 200 à 250 000.

**31.** Kit selon l'une des revendications précédentes, **caractérisé en ce que** le composé Y présente une masse moléculaire en poids (Mw) allant de 200 à 1 000 000, de préférence de 300 à 800 000, de préférence encore de 500 à 250 000.

**32.** Kit selon l'une des revendications précédentes, **caractérisé en ce que** le composé X représente de 0,1 % à 95% en poids par rapport au poids total de la composition le contenant, de préférence de 1% à 90% et mieux de 5% à 80%.

**33.** Kit selon l'une des revendications précédentes, **caractérisé en ce que** le composé Y représente de 0,1% à 95% en poids par rapport au poids total de la composition le contenant, de préférence de 1% à 90% et mieux de 5% à 80%.

**34.** Kit selon l'une des revendications précédentes, **caractérisé en ce que** les composés X et Y sont présents dans les compositions en un ratio molaire X/Y allant de 0,05 à 20, et mieux de 0,1 à 10.

**35.** Kit selon l'une quelconque des revendications précédentes, dans lequel le polymère amphiphile est présent dans une composition dans un rapport massique polymère amphiphile/composé X ouY compris entre 0,01 et 1, en particulier entre 0,05 et 0,2.

**36.** Kit selon l'une quelconque des revendications précédentes, dans lequel les première et seconde compositions sont conditionnées séparément dans un même article de conditionnement.

**37.** Kit selon l'une quelconque des revendications précédentes, dans lequel l'une au moins des première et seconde compositions comprend une phase grasse liquide.

**38.** Kit selon la revendication précédente, dans lequel la phase grasse liquide comprend au moins une huile.

**39.** Kit selon la revendication précédente, dans lequel la ou les huiles sont présentes en une teneur allant de 2 % à 60 % en poids, de préférence de 2 % à 50 % en poids par rapport au poids total de la composition.

**40.** Kit selon l'une quelconque des revendications précédentes, dans lequel l'une au moins des première et seconde compositions comprend une phase aqueuse.

**41.** Kit selon l'une quelconque des revendications précédentes, dans lequel la ou les compositions sont sous forme d'une émulsion, notamment eau dans huile, huile dans eau, eau dans huile dans eau ou huile-dans-eau-dans-huile.

**42.** Kit selon l'une quelconque des revendications précédentes, dans lequel au moins l'une des compositions comprend au moins une matière colorante.

**43.** Kit selon l'une quelconque des revendications précédentes, utile à titre de composition de revêtement de la peau du corps ou du visage, et plus particulièrement de composition de maquillage et /ou de soin de la peau du corps ou du visage.

**44.** Procédé de soin cosmétique et/ou de maquillage de matière(s) kératinique(s) comprenant au moins l'application sur les matières kératiniques (a) d'au moins un polymère amphiphile, (b) d'un ou plusieurs composés X, (c) d'un ou plusieurs composés Y, avec au moins un des composés X et Y étant siliconé et lesdits composés X et Y étant susceptibles de réagir ensemble par une réaction d'hydrosilylation en présence d'un catalyseur, lorsqu'ils sont mis en contact l'un avec l'autre, et (d) d'au moins un catalyseur, les applications (a), (b), (c) et (d) pouvant être simultanées ou séquencées selon un ordre indifférent sous réserve qu'il soit propice à l'interaction desdits composés X et Y.

**45.** Procédé selon la revendication précédente consistant à appliquer sur lesdites matières kératiniques au moins une composition de type émulsion comprenant, dans un milieu physiologiquement acceptable, au moins un composé X, au moins un composé Y, au moins un catalyseur, et au moins un polymère amphiphile.

**46.** Procédé selon la revendication précédente, dans lequel ladite composition est obtenue en mélangeant de façon extemporanée une première composition comprenant au moins le composé X et une seconde composition comprenant au moins le composé Y, l'une au moins des première et seconde composition étant une émulsion et contenant au moins un polymère amphiphile, et au moins l'une des première et seconde composition contenant en outre au moins un catalyseur.

**47.** Procédé cosmétique de maquillage et/ou de soin des matières kératiniques notamment de la peau humaine, comprenant l'application sur lesdites matières kératiniques :

- d'au moins une couche d'une première composition contenant, dans un milieu physiologiquement acceptable, au moins un composé X ;
- d'au moins une couche d'une seconde composition contenant, dans un milieu physiologiquement acceptable, au moins un composé Y,

l'un au moins des composés X et Y étant un composé siliconé, lesdits composés X et Y étant susceptibles de réagir ensemble par une réaction d'hydrosylilation en présence d'un catalyseur, lorsqu'ils sont mis en contact l'un avec l'autre, avec l'une au moins desdites première et seconde compositions étant une émulsion et comprenant au moins un polymère amphiphile, l'une au moins des première et seconde compositions contenant en outre au moins un catalyseur.

**48.** Procédé selon la revendication précédente, dans lequel la première et/ou la seconde compositions sont telles que définies dans l'une quelconque des revendications 1 à 42.

**49.** Procédé selon l'une quelconque des revendications 44 à 48, **caractérisé en ce qu'**il comprend une étape supplémentaire consistant à déposer sur la ou les couches de compositions comprenant X et Y, au moins une couche d'une troisième composition comprenant un polymère filmogène et un milieu solvant organique ou aqueux.

**50.** Kit cosmétique utile pour le soin et/ou le maquillage de matière(s) kératinique(s), notamment de la peau, comprenant au moins deux compositions différentes et conditionnées séparément avec l'une au moins des compositions étant de type émulsion et comprenant au moins un polymère amphiphile, le kit comprenant un ou plusieurs composés X, un ou plusieurs composés Y, et éventuellement au moins un catalyseur, avec au moins un des composés X et Y étant siliconé et lesdits composés X et Y étant susceptibles de réagir ensemble par une réaction de condensation lorsqu'ils sont mis en contact les uns avec les autres, et dans lequel les composés X, Y et le catalyseur, lorsqu'il est présent, ne sont pas présents simultanément dans la même composition.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0196450 A
- GB 2407496 A
- EP 0465744 A
- EP 959066 A
- EP 1057849 A
- WO 9317060 A
- WO 9612754 A
- US 3175993 A
- US 4772675 A
- US 4871827 A
- US 4888380 A
- US 4898910 A
- US 4906719 A
- US 4962174 A
- EP 274961 A
- EP 1552820 A
- FR 2787729
- JP 2295912 A
- US 4234435 A
- US 4708753 A
- US 5129972 A
- US 4931110 A
- GB 2156799 A
- US 4919179 A
- US 5412004 A
- US 5811487 A


### Littérature non-brevet citée dans la description


- **DONALD A. TOMALIA et al.** *Angewandte Chemie, Int. Engl. Ed.,* vol. 29 (2), 138-175
- **KUSABE.M.** *Pitture e Verniei - European Coating,* 2005, vol. 12-B, 43-49
- **PROBSTER, M.** *Adhesion-Kleben & Dichten,* 2004, vol. 481 (1-2), 12-14
- **LANDON, S.** *Pitture e Verniei,* 1997, vol. 73 (11), 18-24
- **HUANG, MOWO.** *Pitture e Verniei,* 2000, vol. 5, 61-67
- **ALLGAIER ; POPPE ; WILLNER ; RICHTER.** *Macromolecules,* 1997, vol. 30, 1582-1586